(19) 
**Europäisches Patentamt**
**European Patent Office**
**Office européen des brevets**

(11) **EP 4 379 356 A1**

(12) **EUROPEAN PATENT APPLICATION**
published in accordance with Art. 153(4) EPC

(43) Date of publication:
**05.06.2024 Bulletin 2024/23**

(21) Application number: **22849340.9**

(22) Date of filing: **20.07.2022**

(51) International Patent Classification (IPC):
*G01N 21/64* (2006.01)   *B22F 9/00* (2006.01)
*B22F 9/24* (2006.01)   *C23C 18/31* (2006.01)
*C23C 18/44* (2006.01)   *C23C 28/00* (2006.01)
*G01N 21/41* (2006.01)

(52) Cooperative Patent Classification (CPC):
**B22F 1/054; B22F 1/16; B22F 1/18; B22F 9/00;
B22F 9/24; B82Y 20/00; C23C 18/31; C23C 18/44;
C23C 18/52; C23C 20/08; C23C 28/00;
G01N 21/41; G01N 21/64**

(86) International application number:
**PCT/JP2022/028198**

(87) International publication number:
**WO 2023/008278 (02.02.2023 Gazette 2023/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.07.2021   JP 2021123210
23.12.2021   JP 2021209090**

(71) Applicant: **Sumitomo Chemical Company Limited
Tokyo 103-6020 (JP)**

(72) Inventor: **FUKUURA, Tomohiro
Osaka-shi, Osaka 554-8558 (JP)**

(74) Representative: **Vossius & Partner
Patentanwälte Rechtsanwälte mbB
Siebertstrasse 3
81675 München (DE)**

(54) **COMPOSITE PARTICLES AND PRODUCTION METHOD THEREFOR, AND SENSOR ELEMENT**

(57)   The present invention provides a novel structure that can be used for a sensor element or the like and is useful as an enhancing element to enhance the emission of a luminescent substance. Provided is a composite particle comprising a core particle, and a metal-based particle assembly layer arranged on at least a portion of a surface of the core particle, wherein the metal-based particle assembly layer comprises a plurality of metal-based particles arranged apart from each other.

[Figure 1]

Processed by Luminess, 75001 PARIS (FR)

**Description**

Technical Field

The present invention relates to a composite particle, a method for producing the same, and a sensor element.

Background Art

A technique exploiting localized plasmon resonance phenomenon of metal nanoparticles for enhanced fluorescence is known (for example, Patent Literature 1). Non Patent Literature 1 indicates a study on localized plasmon resonance of silver nanoparticles.

Citation List

Patent Literature

Patent Literature 1: Japanese Patent Laid-Open No. 8-271431

Non Patent Literature

Non Patent Literature 1: T. Fukuura and M. Kawasaki, "Long Range Enhancement of Molecular Fluorescence by Closely Packed Submicro-scale Ag Islands", e-Journal of Surface Science and Nanotechnology, 2009, 7, 653

Summary of Invention

Technical Problem

One object of the present invention is to provide a novel structure that can be used in a sensor element or the like and is useful as an enhancing element to enhance the emission of a luminescent substance.

Another object of the present invention is to provide a sensor element comprising such a structure.

Solution to Problem

The present invention provides the following composite particle, method for producing the same, and sensor element.

[1] A composite particle comprising:

a core particle; and
a metal-based particle assembly layer arranged on at least a portion of a surface of the core particle,
wherein the metal-based particle assembly layer comprises a plurality of metal-based particles arranged apart from each other.

[2] The composite particle according to [1], wherein the plurality of metal-based particles have an average particle diameter of 5 nm or more and 1600 nm or less.
[3] The composite particle according to [1] or [2], wherein the plurality of metal-based particles are each arranged so that an average distance between metal-based particles adjacent to each other is 1 nm or more and 150 nm or less.
[4] The composite particle according to any one of [1] to [3], wherein a standard deviation of the average distance is 50 nm or less.
[5] The composite particle according to any one of [1] to [4], wherein the surface of the core particle comprises a curved surface.
[6] The composite particle according to [5], wherein the metal-based particle assembly layer is arranged at least on the curved surface.
[7] The composite particle according to any one of [1] to [6], wherein the core particle has a particle diameter of 0.3 μm or more and less than 10 μm.
[8] The composite particle according to any one of [1] to [7], wherein the metal-based particle assembly layer is arranged on an entire surface of the core particle.
[9] The composite particle according to any one of [1] to [8], further comprising a protective layer that covers at least surfaces of the plurality of metal-based particles.

[10] The composite particle according to [9], wherein the protective layer comprises an insulating material.

[11] The composite particle according to [9] or [10], wherein the protective layer has a thickness of 3 nm or more and 200 nm or less.

[12] The composite particle according to any one of [9] to [11], wherein the protective layer covers entire surfaces of the core particle and the metal-based particles.

[13] The composite particle according to any one of [1] to [12], which is for a sensor element.

[14] A method for producing a composite particle,

the composite particle comprising:

a core particle; and

a metal-based particle assembly layer arranged on at least a portion of a surface of the core particle,

wherein the metal-based particle assembly layer comprises a plurality of metal-based particles arranged apart from each other, and

wherein the method comprises the step of growing the metal-based particles on the surface of the core particle.

[15] The method for producing a composite particle according to [14], further comprising, after the step of growing, a step of performing a heat treatment.

[16] The method for producing a composite particle according to [14] or [15], further comprising, after the step of growing, a step of forming a protective layer that covers at least surfaces of the plurality of metal-based particles.

[17] A composite particle comprising:

a core particle;

a metal-based material layer arranged on at least a portion of a surface of the core particle; and

a protective layer that covers a surface of the metal-based material layer.

[18] The composite particle according to [17], wherein the protective layer comprises an insulating material.

[19] The composite particle according to [17] or [18], wherein the protective layer has a thickness of 3 nm or more and 200 nm or less.

[20] A sensor element used for detecting an analyte, comprising:

a substrate;

a composite particle arranged on the substrate; and

a capturing portion that is arranged on the composite particle and has a capturing substance that specifically binds to the analyte,

wherein the composite particle comprises:

a core particle; and

a metal-based particle assembly layer arranged on at least a portion of a surface of the core particle,

wherein the metal-based particle assembly layer comprises a plurality of metal-based particles arranged apart from each other.

[21] The sensor element according to [20], wherein the composite particle further comprises a protective layer that covers at least surfaces of the plurality of metal-based particles, and

the capturing portion is arranged on the protective layer.

Advantageous Effects of Invention

There is provided a novel structure that can be used for a sensor element or the like and is useful as an enhancing element to enhance the emission of a luminescent substance. There is also provided a sensor element comprising such a structure.

Brief Description of Drawings

[Figure 1] Figure 1 is a plan view schematically showing one example of the composite particle according to the present invention.

[Figure 2] Figure 2 is a cross-sectional view schematically showing one example of the composite particle according to the present invention.

[Figure 3] Figure 3 is a cross-sectional view schematically showing another example of the composite particle according to the present invention.

[Figure 4] Figure 4 is a schematic diagram for illustrating one example of the metal-based particle growth step in a plating method.

[Figure 5] Figure 5 is a cross-sectional view schematically showing another example of the sensor element according to the present invention.

[Figure 6] Figure 6 shows a SEM image (at a scale of 11000 times) of the composite particles (after the heat treatment step) obtained in Example 1.

[Figure 7] Figure 7 shows a SEM image (at a scale of 10000 times) of the composite particles including a protective layer obtained in Example 2.

[Figure 8] Figure 8 shows an image obtained by fluorescence intensity measurement for the measurement sample 1.

[Figure 9] Figure 9 shows an image obtained by fluorescence intensity measurement for the measurement sample 2.

[Figure 10] Figure 10 shows a SEM image (at a scale of 6000 times) of the composite particles obtained in Example 3.

[Figure 11] Figure 11 shows a SEM image (at a scale of 35000 times) of the composite particles obtained in Example 3.

[Figure 12] Figure 12 shows a SEM image (at a scale of 20000 times) of the composite particles obtained in Example 4.

[Figure 13] Figure 13 shows an image obtained by fluorescence intensity measurement for the measurement sample 3.

[Figure 14] Figure 14 shows an image obtained by fluorescence intensity measurement for the measurement sample 4.

[Figure 15] Figure 15 shows a SEM image (at a scale of 7000 times) of the composite particles including a protective layer obtained in Example 5.

[Figure 16] Figure 16 shows a SEM image (at a scale of 30000 times) of the composite particles including a protective layer obtained in Example 5.

[Figure 17] Figure 17 shows a STEM cross-sectional image (at a scale of 100000 times) of the composite particles including a protective layer obtained in Example 5.

[Figure 18] Figure 18 shows a SEM image (at a scale of 90000 times) of the composite particles obtained in Example 7.

[Figure 19] Figure 19 shows a SEM image (at a scale of 20000 times) of the composite particles obtained in Example 8.

[Figure 20] Figure 20 shows a SEM image (at a scale of 30000 times) of layered products containing the composite particles obtained in Example 9.

[Figure 21] Figure 21 shows a SEM image (at a scale of 35000 times) of layered products containing the composite particles obtained in Example 10.

[Figure 22] Figure 22 shows a SEM image (at a scale of 35000 times) of layered products containing the composite particles obtained in Example 11.

Description of Embodiments

<Composite Particle>

(1) Summary

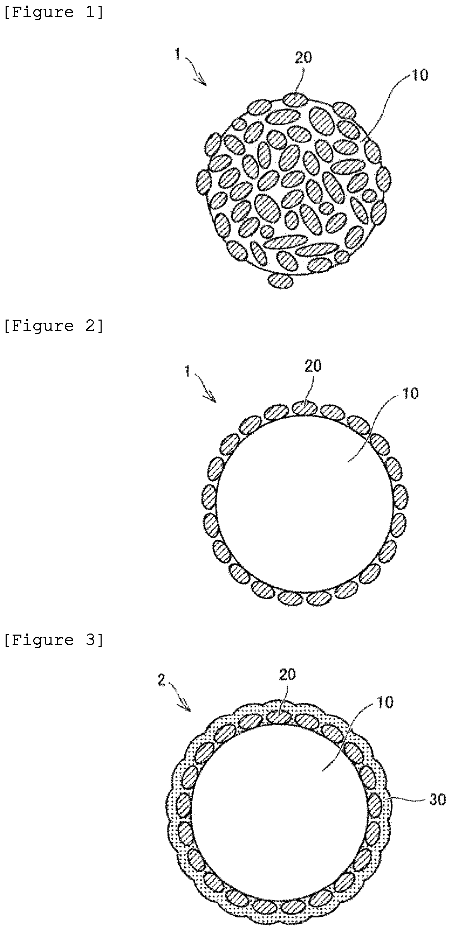

Figure 1 is a plan view schematically showing one example of the composite particle according to the present invention (hereinafter also simply referred to as the "composite particle"), and Figure 2 is a cross-sectional view schematically showing one example of the composite particle. A composite particle 1 comprises a core particle 10 and a metal-based particle assembly layer arranged on at least a portion of a surface of the core particle 10. The metal-based particle assembly layer is an assembly of a plurality of metal-based particles 20 supported on the core particle 10, and is a layer comprising the plurality of metal-based particles 20 arranged apart from each other.

In the example shown in Figures 1 and 2, the metal-based particle assembly layer is arranged on an entire surface of the core particle 10. It is noted, however, that because the plurality of metal-based particles 20 constituting the metal-based particle assembly layer are spaced apart from each other, the metal-based particle assembly layer includes regions where the metal-based particles 20 are absent, and the surface of the core particle 10 is exposed through these regions.

The metal-based particle assembly layer formed on the core particle is a plasmonic structure. "Plasmonic structure" refers to a structure capable of exhibiting localized plasmon resonance. Plasmon is a compressional wave of free electrons generated by collective oscillation of the free electrons in a structure.

Because of the localized plasmon resonance, the composite particle can enhance, for example, the intensity of emission (for example, fluorescence) from a luminescent substance that labels an analyte. Thus, the composite particle is suitable for use as an emission enhancing element for various sensor elements. By applying the composite particle to a sensor element, the sensitivity, quantitative accuracy and/or reproducibility (stability) of quantitative results of the sensor element can be improved. One suitable example of the sensor element is a biosensor element.

The composite particle can exhibit the following characteristics [a] and [b]. These characteristics are believed to be achieved by interactions between localized plasmons exhibited by the plurality of metal-based particles constituting the metal-based particle assembly layer.

[a] The range of the effect of plasmon resonance (the range where the emission enhancing effect of plasmon is effective) exhibited by the metal-based particle assembly layer is broad, and even the emission from the luminescent substance present in the range of, for example, several hundred nm (for example, 200 nm) from the surface of the metal-based particle assembly layer can be enhanced.

[b] The metal-based particle assembly layer exhibits intense plasmon resonance, and hence, a high emission enhancing effect can be achieved.

Regarding the characteristic [a], the composite particle can enhance the emission of the luminescent substance arranged at a position, for example, 10 nm or more away, further several ten nm (for example 20 nm, 30 nm or 40 nm) or more away, and still further 100 nm or more, or 200 nm or more away, from the metal-based particle assembly layer. Regarding the characteristic [b], the intensity of plasmon resonance exhibited by the composite particle is not that of a simple sum total of localized plasmon resonances exhibited by the individual metal-based particles at a specific wavelength, but rather, is higher than that. In the composite particle, intense plasmon resonance is achieved by interactions of the individual metal-based particles with each other. This intense plasmon resonance is believed to be achieved by interactions between localized plasmons of the metal-based particles.

In general, when a plasmonic structure is subjected to absorption spectrum measurement by absorptiometry, a plasmon resonance peak (hereinafter also referred to as a "plasmon peak") is observed as a peak at the longest wavelength in the ultraviolet to visible light region. The intensity of the plasmon resonance of the plasmonic structure can be evaluated based on the magnitude of the absorbance at the maximum wavelength of the plasmon peak. The larger the value of absorbance, the higher the intensity of the plasmon resonance tends to be.

The absorption spectrum of the plasmonic structure can be measured by absorptiometry. Specifically, the absorption spectrum is obtained as follows: Incident light in the ultraviolet to visible light region is directed from the back surface side of the glass substrate layered with the metal-based particle assembly (i.e., the side opposite to the metal-based particle assembly) in a direction perpendicular to the substrate surface, and intensity $I$ of the transmitted light omnidirectionally transmitted toward the metal-based particle assembly is measured with an integrating sphere spectrophotometer. On the other hand, the same incident light as described above is directed in a direction perpendicular to the surface of a substrate not layered with the metal-based particle assembly, which has the same thickness and the same material as those of the substrate of the measurement sample, and intensity $I_0$ of transmitted light omnidirectionally transmitted through the side opposite to the incident surface is measured with the integrating sphere spectrophotometer. In this case, the absorbance along the vertical axis in the absorption spectrum is represented by the following expression:

$$\texttt{Absorbance} = -\log_{10}(\texttt{I}/\texttt{I}_0)$$

The absorption spectrum can be measured using a common spectrophotometer.

To measure the maximum wavelength and the absorbance at the maximum wavelength of the plasmon peak at the longest wavelength in the ultraviolet to visible light region, absorption spectrum measurement may be performed using an objective lens and a spectrophotometer to narrow the field of view.

(2) Core Particle

The core particle is a particle constituting the core of the composite particle. The shape of the core particle is not limited to a particular shape, and may be a shape in which the surface comprises a curved surface or may be a shape in which the surface does not comprise a curved surface.

Examples of the shape in which the surface comprises a curved surface include a spherical shape and a spheroidal shape. Examples of the shape in which the surface does not comprise a curved surface include a polyhedral shape. A portion of the surface of the core particle may be formed by a curved surface, with the remaining portion being formed by a flat surface. Alternatively, the core particle may have an irregular shape.

The core particle is preferably spherical or substantially spherical.

The core particle has a particle diameter of, for example, 0.3 $\mu$m or more and less than 10 $\mu$m, preferably 0.5 $\mu$m or more and 8 $\mu$m or less, more preferably 1 $\mu$m or more and 5 $\mu$m or less, still more preferably 1 $\mu$m or more and

3 μm or less. The present invention also relates to a group of composite particles that is a collection of a plurality of composite particles. In the group of composite particles, the particle diameter of the core particle may mean the average particle diameter of a group of core particles (a collection of a plurality of core particles).

The particle diameter of the core particle is measured according to the following method: The scanning electron microscope "JSM-5500" produced by JEOL Ltd. or its equivalent is used to obtain a SEM image including a core particle as observed from directly above a substrate (such as a glass substrate) to which the core particle is adhered. In the SEM image, five tangential diameters are drawn at random in the core particle image (note that all the straight lines serving as the tangential diameters can pass through only the inside of the core particle image, and one of these lines is a straight line that passes through only the inside of the core particle and can be drawn the longest), and the average value thereof (average tangential diameter) is obtained as the particle diameter of the core particle. The tangential diameter is defined as a perpendicular line connecting a distance defined by two parallel lines sandwiching the particle contour (projected image) in contact therewith (see the Nikkan Kogyo Shimbun, Ltd., "Particle Measurement Technique," 1994, page 5).

The average particle diameter is measured according to the following method: The scanning electron microscope "JSM-5500" produced by JEOL Ltd. or its equivalent is used to obtain a SEM image including a group of core particles as observed from directly above a substrate (such as a glass substrate) to which the group of core particles are adhered. In the SEM image, 10 core particles are selected at random, five tangential diameters are drawn at random in each core particle image (note that all the straight lines serving as the tangential diameters can pass through only the inside of the core particle image, and one of these lines is a straight line that passes through only the inside of the core particle and can be drawn the longest), the average value thereof (average tangential diameter) is obtained, and average tangential diameters are obtained for all the 10 selected core particles. The tangential diameter is defined as set forth above. A quarter length of the average tangential diameter of each core particle is defined as the "edge region length" of each core particle. The range of the "edge region length" from the contour line toward the inside of each core particle, on the SEM image of the core particle, is defined as the "edge region" of each core particle. Then, if any core particle of the 10 core particles overlaps even partially with the edge region of another core particle, the core particle is discarded, and then an insufficient number of core particles are newly selected at random, and average tangential diameters are obtained in the same manner as above. This procedure is repeated as required to obtain average tangential diameters for 10 core particles that do not overlap with the edge region. The average value of the 10 average tangential diameters thus obtained is obtained as the average particle diameter.

The particle diameter of the core particle contained in the composite particle can be obtained based on the following expression:

particle diameter of the core particle = (average particle diameter of the composite particles) - 2 × {(average height of the metal-based particles) + (thickness of the protective layer)}

The average particle diameter of the composite particles can be measured in the same manner as described for the average particle diameter of the core particles. The method of measuring the average height of the metal-based particles and the method of measuring the thickness of the protective layer will be described below.

While the material of the core particle is not limited to a particular material, the core particle is preferably non-conductive, in view of increasing the function of the composite particle as an emission enhancing element, and thus, the core particle preferably contains an insulating material. Examples of the insulating material include inorganic insulating materials, such as silica, titania, alumina, and silicon nitride; and organic insulating materials, such as resin materials (for example, polystyrene, acrylic resin, cellulose nanofibers, paper, nitrocellulose, and epoxy resin). Other examples may include semiconductors such as silicon. When it is desired to obtain a composite particle containing a core particle that is spherical or substantially spherical, the material of the core particle is preferably silica, which facilitates industrial production of spherical or substantially spherical particles.

The core particle may be composed of two or more materials. The core particle may have a single-layer structure or a multilayer structure. For example, the core particle may have a core formed of a metal material and a shell formed of an insulating material to thereby exhibit non-conductivity. Examples include silicon with an insulating film; and a metal (such as aluminum, copper, chromium, or a composite thereof) with an insulating film.

(3) Metal-Based Particle Assembly Layer

The metal-based particle assembly layer is arranged on at least a portion of a surface of the core particle, and may be arranged over an entire surface of the core particle. The plurality of metal-based particles constituting the metal-based particle assembly layer are spaced apart from each other.

The metal-based particles is formed of a material capable of plasmon resonance in the ultraviolet to visible light region. The material capable of plasmon resonance in the ultraviolet to visible light region means a material that, when in the form of nanoparticles or an assembly thereof, exhibits a plasmon peak appearing in the ultraviolet to visible light region in absorption spectrum measurement by absorptiometry.

Examples of metal-based materials capable of plasmon resonance in the ultraviolet to visible light region include noble metals, such as gold, silver, copper, platinum, and palladium; metals other than noble metals, such as aluminum and tantalum; alloys containing metals selected from the noble metals and the metals other than noble metals; and metal compounds (such as metal oxides and metal salts) containing metals selected from the noble metals and the metals other than noble metals. Among these, preferred metal-based materials capable of plasmon resonance in the ultraviolet to visible light region are noble metals, such as gold, silver, copper, platinum, and palladium, and more preferred is silver, because it is inexpensive and has small absorption (or has a small imaginary part of the dielectric function at visible light wavelengths).

The plurality of metal-based particles constituting the metal-based particle assembly layer may have an average particle diameter of 5 nm or more, preferably 30 nm or more, more preferably 100 nm or more, still more preferably 200 nm or more, even more preferably 250 nm or more, in view of effectively achieving the above-described effects [a] and [b]. The plurality of metal-based particles constituting the metal-based particle assembly layer may have an average particle diameter of, for example, 1600 nm or less, preferably 800 nm or less, more preferably 550 nm or less, still more preferably 450 nm or less, even more preferably 400 nm or less, particularly preferably 350 nm or less, in view of effectively achieving the above-described effects [a] and [b].

The average particle diameter of the metal-based particles is preferably selected appropriately according to the type of metal-based material constituting the metal-based particles.

The average particle diameter of the plurality of metal-based particles refers to the average value of the particle diameter obtained as follows: In a SEM image including a composite particle as observed from directly above a substrate (such as a glass substrate) to which the composite particle is adhered, six metal-based particles are selected at random. In each metal-based particle image, five tangential diameters are drawn at random (note that all the straight lines serving as the tangential diameters can pass through only the inside of the metal-based particle image, and one of these lines is a straight line that passes through only the inside of the metal-based particle and can be drawn the longest). When the average value thereof (this average value is hereinafter also referred to as the "average tangential diameter") is obtained as the particle diameter of each metal-based particle, the average particle diameter of the plurality of metal-based particles is the average value of the particle diameters for the six selected metal-based particles. The tangential diameter is defined as set forth above.

The method of measuring the average particle diameter is more specifically described as follows: Initially, the scanning electron microscope "JSM-5500" produced by JEOL Ltd. or its equivalent is used to measure a SEM image. Then, the free image processing software "ImageJ" produced by U.S. National Institutes of Health is used to read in the obtained image at 1280 horizontal pixels $\times$ 960 vertical pixels. Then, the random number generator function "RANDBETWEEN" of the spreadsheet software "Excel" produced by Microsoft Corporation is used to obtain 6 random numbers $(x_1, x_2, x_3, x_4, x_5,$ and $x_6)$ from 1 to 1280 and 6 random numbers $(y_1, y_2, y_3, y_4, y_3,$ and $y_6)$ from 1 to 960. From the respective sets of 6 random numbers thus obtained, 6 sets of random number combinations $(x_1, y_1)$, $(x_2, y_2)$, $(x_3, y_3)$, $(x_4, y_4)$, $(x_5, y_5)$, and $(x_6, y_6)$ are obtained. Using the values of the random numbers generated from 1 to 1280 as x-coordinates and the values of the random numbers generated from 1 to 960 as y-coordinates, 6 sets of coordinate points $(x_1, y_1)$, $(x_2, y_2)$, $(x_3, y_3)$, $(x_4, y_4)$, $(x_5, y_5)$, and $(x_6, y_6)$ are obtained. Then, for each of the total of six metal-based particle images including the coordinate points, the above-described average tangential diameter is obtained, and then the average particle diameter is obtained as the average value of the average tangential diameters for the six metal-based particles. Here, if at least any one of the six coordinate points that are the six sets of random number combinations is not included in a metal-based particle image, or if two or more coordinate points are included in an identical metal-based particle, then these random number combinations are discarded, and random number generation is repeated until all the six coordinate points are included in different metal-based particle images. Here, "edge region" in the core particle described above is also obtained from the same image. Then, if any metal-based particle of the six metal-based particles overlaps even partially with the edge region in the core particle on which each of the metal-based particles is arranged, the random number combinations are discarded, and random number generation is repeated until all the six coordinate points are included in different metal-based particle images, and there is no metal-based particle that overlaps even partially with the edge region in the core particle on which each of the metal-based particles is arranged. The average tangential diameter is then obtained in the same manner as above. This procedure is repeated as required to obtain average tangential diameters for six metal-based particles that do not overlap with the edge region in the core particle. The average value of the six average tangential diameters thus obtained is obtained as the average particle diameter.

Usually, the average particle diameter of the metal-based particles is smaller than the particle diameter of the core particle on which they are supported. The ratio of the particle diameter of the core particle to the average particle diameter of the metal-based particles is, for example, 2 or more and 10000 or less, preferably 4 or more and 5000 or less, and more preferably 6 or more and 1000 or less.

In the metal-based particle assembly layer, the plurality of metal-based particles are each arranged so that an average distance between metal-based particles adjacent to each other (hereinafter also referred to as the "average interparticle distance") is 1 nm or more and 150 nm or less. When the plurality of metal-based particles are arranged at this average interparticle distance, intense plasmon resonance can be obtained and simultaneously, the effect of extending the range of the effect of plasmon resonance can be increased.

The average interparticle distance is preferably 1 nm or more and 120 nm or less, more preferably 1 nm or more and 100 nm or less, still more preferably 1 nm or more and 80 nm or less, even more preferably 1 nm or more and 60 nm or less, and particularly preferably 1 nm or more and 40 nm or less, in view of effectively achieving the above-described effects [a] and [b]. If the average interparticle distance is less than 1 nm, electron transfer between the particles attributed to the Dexter mechanism occurs, which disadvantageously deactivates localized plasmon.

The average interparticle distance is obtained as follows: In a SEM image including a composite particle as observed from directly above a substrate (such as a glass substrate) to which the composite particle is adhered, six metal-based particles are selected at random, and for each selected metal-based particle, an interparticle distance from an adjacent metal-based particle is obtained, and the average value of interparticle distances of these six metal-based particles is obtained as the average interparticle distance. The interparticle distance from an adjacent metal-based particle is obtained as follows: A distance from every adjacent metal-based particle (minimum distance between the surfaces of adjacent metal-based particles) is measured, and such measurements are averaged to obtain the interparticle distance from an adjacent metal-based particle.

The method of measuring the average interparticle distance is more specifically described as follows: Initially, the scanning electron microscope "JSM-5500" produced by JEOL Ltd. or its equivalent is used to measure a SEM image. Then, the free image processing software "ImageJ" produced by U.S. National Institutes of Health is used to read in the obtained image at 1280 horizontal pixels × 960 vertical pixels. Then, the random number generator function "RANDBETWEEN" of the spreadsheet software "Excel" produced by Microsoft Corporation is used to obtain 6 random numbers ($x_1$ to $x_6$) from 1 to 1280 and 6 random numbers ($y_1$ to $y_6$) from 1 to 960. From the respective sets of 6 random numbers thus obtained, 6 sets of random number combinations ($x_1$, $y_1$) to ($x_6$, $y_6$) are obtained. Using the values of the random numbers generated from 1 to 1280 as x-coordinates and the values of the random numbers generated from 1 to 960 as y-coordinates, 6 sets of coordinate points ($x_1$, $y_1$) to ($x_6$, $y_6$) are obtained. Then, for each of the total of 6 metal-based particle images including the coordinate points, an interparticle distance from a metal-based particle adjacent to that metal-based particle is obtained, and then the average interparticle distance is obtained as the average value of interparticle distances from adjacent metal-based particles for the 6 metal-based particles. Here, if at least any one of the six coordinate points that are the six sets of random number combinations is not included in a metal-based particle image, or if two or more coordinate points are included in an identical metal-based particle, then these random number combinations are discarded, and random number generation is repeated until all the six coordinate points are included in different metal-based particle images. Here, "edge region" in the core particle described above is also obtained from the same image. Then, if any metal-based particle of the six metal-based particles overlaps even partially with the edge region in the core particle on which each of the metal-based particles is arranged, the random number combinations are discarded, and random number generation is repeated until all the six coordinate points are included in different metal-based particle images, and there is no metal-based particle that overlaps even partially with the edge region in the core particle on which each of the metal-based particles is arranged. The interparticle distance is then obtained in the same manner as above. This procedure is repeated as required to obtain interparticle distances for six metal-based particles that do not overlap with the edge region in the core particle. The average value of the interparticle distances of the six metal-based particles thus obtained is obtained as the average interparticle distance.

In the metal-based particle assembly layer, the plurality of metal-based particles are preferably arranged so that a standard deviation of the average interparticle distance is 50 nm or less. When the metal-based particles are arranged so that the standard deviation falls in this range, intense plasmon resonance can be obtained and simultaneously, the effect of extending the range of the effect of plasmon resonance can be increased.

The standard deviation of the average interparticle distance is preferably 40 nm or less, more preferably 30 nm or less, still more preferably 25 nm or less, and even more preferably 20 nm or less, in view of effectively achieving the above-described effects [a] and [b]. The standard deviation of the average interparticle distance is preferably 0.1 nm or more, more preferably 0.2 nm or more, and still more preferably 0.3 nm or more, in view of effectively achieving the above-described effects [a] and [b].

The standard deviation of the average interparticle distance is defined as follows: In a SEM image including a composite particle as observed from directly above a substrate (such as a glass substrate) to which the composite particle is adhered, one metal-based particle is initially selected at random, and for that metal-based particle, an interparticle distance from an adjacent metal-based particle is obtained. A distance from every adjacent metal-based particle (minimum distance between their surfaces) is measured, and such measurements are averaged to obtain the interparticle distance from an adjacent metal-based particle. In the above-described SEM image, 5 metal-based particles different from the above-described one metal-based particle are selected at random, and interparticle distances from adjacent metal-based particles are obtained for these 5 metal-based particles in the same manner as above. The standard deviation of the interparticle distances from adjacent metal-based particles for the total of 6 metal-based particles thus obtained is defined as the standard deviation of the average interparticle distance.

The method of measuring the standard deviation of the average interparticle distance is more specifically described as follows: Initially, the scanning electron microscope "JSM-5500" produced by JEOL Ltd. or its equivalent is used to measure a SEM image. Then, the free image processing software "ImageJ" produced by U.S. National Institutes of Health is used to read in the obtained image at 1280 horizontal pixels × 960 vertical pixels. Then, the random number generator function "RANDBETWEEN" of the spreadsheet software "Excel" produced by Microsoft Corporation is used to obtain 6 random numbers ($x_1$ to $x_6$) from 1 to 1280 and 6 random numbers ($y_1$ to $y_6$) from 1 to 960. From the respective sets of 6 random numbers thus obtained, 6 sets of random number combinations ($x_1$, $y_1$) to ($x_6$, $y_6$) are obtained. Using the values of the random numbers generated from 1 to 1280 as x-coordinates and the values of the random numbers generated from 1 to 960 as y-coordinates, 6 sets of coordinate points ($x_1$, $y_1$) to ($x_6$, $y_6$) are obtained. Then, for each of the total of 6 metal-based particle images including the coordinate points, an interparticle distance from a metal-based particle adjacent to that metal-based particle is obtained, and then the standard deviation of the average interparticle distance is obtained as the standard deviation of interparticle distances from adjacent metal-based particles for the 6 metal-based particles. Here, if at least any one of the six coordinate points that are the six sets of random number combinations is not included in a metal-based particle image, or if two or more coordinate points are included in an identical metal-based particle, then these random number combinations are discarded, and random number generation is repeated until all the six coordinate points are included in different metal-based particle images. Here, "edge region" in the core particle described above is also obtained from the same image. Then, if any metal-based particle of the six metal-based particles overlaps even partially with the edge region in the core particle on which each of the metal-based particles is arranged, the random number combinations are discarded, and random number generation is repeated until all the six coordinate points are included in different metal-based particle images, and there is no metal-based particle that overlaps even partially with the edge region in the core particle on which each of the metal-based particles is arranged. The interparticle distance is then obtained in the same manner as above. This procedure is repeated as required to obtain interparticle distances for six metal-based particles that do not overlap with the edge region in the core particle. The standard deviation of the average interparticle distance is obtained as the standard deviation of interparticle distances from adjacent metal-based particles for the six metal-based particles.

The plurality of metal-based particles constituting the metal-based particle assembly layer preferably have an average height of 5 nm or more and 500 nm or less, more preferably 10 nm or more and 300 nm or less, still more preferably 30 nm or more and 200 nm or less, even more preferably 50 nm or more and 150 nm or less, particularly preferably 55 nm or more and 150 nm or less, in view of effectively achieving the above-described effects [a] and [b].

The average height of the plurality of metal-based particles is measured according to the following method: The scanning electron microscope "JSM-5500" produced by JEOL Ltd. or its equivalent is used to obtain a cross-sectional image of the composite particle. In the obtained cross-sectional image, 10 points are selected at random on an outer contour surface of the particle formed of the core particle and the metal-based particle assembly layer (the composite particle not having the protective layer). Then, a straight line is drawn to be shortest from each of the 10 points to an outer surface of the core particle, and the average value of the lengths of the 10 straight lines thus obtained is obtained as the average height of the metal-based particles.

The plurality of metal-based particles constituting the metal-based particle assembly layer preferably have an aspect ratio of 1 or more and 8 or less, more preferably 1 or more and 7 or less, still more preferably 1.5 or more and 7 or less, even more preferably 1.5 or more and 6 or less, in view of effectively achieving the above-described effects [a] and [b].

The aspect ratio is defined as the ratio of the above-described average particle diameter to the above-described average height (i.e., average particle diameter/average height). While the metal-based particles may have a spherical shape, they preferably have a flat shape with an aspect ratio of above 1, in view of effectively achieving the above-described effects [a] and [b].

The metal-based particles preferably have a smooth curved surface, and particularly preferably have a flat shape whose surface is a smooth curved surface, in view of exciting highly effective plasmon. However, the metal-based particles may have some minute recesses and projections (roughness) on the surface, and may in that sense have an amorphous shape.

For a single composite particle, the number of the metal-based particles contained in the metal-based particle assembly layer is usually 6 or more, and preferably 30 or more. When the metal-based particle assembly layer is formed to contain six or more metal-based particles, intense plasmon resonance and an extended range of the effect of plasmon resonance can be easily achieved because of interactions between localized plasmons of the metal-based particles. For a single composite particle, the number of the metal-based particles contained in the metal-based particle assembly layer may be, for example, 50 or more, further 1000 or more, and still further 10000 or more.

The metal-based particles preferably have a number density of 7 or more, more preferably 15 or more, per

$\mu m^2$ of surface area of the core particle, in view of facilitating achieving intense plasmon resonance and an extended range of the effect of plasmon resonance.

The composite particle in which the metal-based particle assembly layer is arranged on the surface of the core particle and the composite particle in which the below-described protective layer is further arranged on the surface of the particle have surface recesses and projections or may be formed to have surface recesses and projections. The composite particle with surface recesses and projections has a larger surface area. Thus, when the composite particle is applied to a sensor element, a larger amount of the capturing substance and hence the analyte can be bound to the surface of the composite particle. This is advantageous for improving the detection sensitivity and detection accuracy of the sensor element.

The metal-based particle assembly layer layered on the surface of the core particle is preferably non-conductive as a layer, in view of facilitating achieving intense plasmon resonance and an extended range of the effect of plasmon resonance. From this viewpoint, the surface of the core particle on which the metal-based particles are arranged is preferably also non-conductive. The metal-based particles themselves may be conductive.

In the composite particle according to one embodiment, the core particle comprises a curved surface, and the metal-based particle assembly layer is arranged at least on the curved surface. In the composite particle according to another embodiment, the core particle is spherical or substantially spherical, and the metal-based particle assembly layer is arranged on the entire surface of the core particle.

(4) Protective Layer

The composite particle may further comprise a protective layer 30, as in a composite particle 2 shown in Figure 3, which is a cross-sectional view schematically showing another example of the composite particle. The composite particle 2 comprises a core particle 10, a metal-based particle assembly layer (assembly of a plurality of metal-based particles 20) arranged on at least a portion of a surface of the core particle 10, and the protective layer 30 that covers at least surfaces of the plurality of metal-based particles 20.

Formation of the protective layer is advantageous in the following respects:

[A] When the composite particle is used as an emission enhancing element to enhance the intensity of emission from a luminescent substance that labels the analyte, if the luminescent substance is in direct contact with the metal-based particles of the composite particle, quenching due to electron tunneling from the luminescent substance to the metal-based particles may occur, resulting in a decrease in the enhancement effect. The protective layer provided on the metal-based particles ensures that the luminescent substance and the metal-based particles are spaced apart, which can inhibit quenching.

[B] The stability (such as oxidation resistance) and environmental stability (for example, light resistance, humidity resistance, and heat resistance) of the composite particle (the core particle and/or the metal-based particles) can be increased.

From the viewpoint of [A] above, the protective layer may be formed to cover at least the surfaces of the plurality of the metal-based particles constituting the metal-based particle assembly layer; however, as shown in Figure 3, the protective layer may also be formed to cover an entire surface including the surface of the core particle and the surfaces of the plurality of metal-based particles.

From the viewpoint of [A] above, the material of the protective layer is preferably a non-conductive material, i.e., an insulating material. Examples of the insulating material include inorganic insulating materials, such as spin-on-glass (SOG; for example, a material containing an organosiloxane material), $SiO_2$, SiN, TiOz, $Al_2O_3$, and $Si_3N_4$; and organic insulating materials, such as resin materials (for example, polystyrene, acrylic resin, and epoxy resin). The protective layer may be composed of two or more materials. The protective layer may have a single-layer structure or a multilayer structure.

While the thickness of the protective layer is not limited to a particular thickness, it is, for example, 3 nm or more, and preferably 10 nm or more. When the composite particle is used as an emission enhancing element in a sensor element for capturing an analyte labeled with a luminescent substance onto the protective layer and analyzing the analyte, the thickness of the protective layer is, for example, 200 nm or less, preferably 150 nm or less, more preferably 100 nm or less, still more preferably 80 nm or less, and even more preferably 50 nm or less, in view of effectively enhancing the emission from the luminescent substance.

The thickness of the protective layer is measured according to the following method: The scanning electron microscope "JSM-5500" produced by JEOL Ltd. or its equivalent is used to obtain a cross-sectional image of the composite particle. In the obtained cross-sectional image, 10 points are selected at random on an outer contour surface of the particle formed of the core particle and the metal-based particle assembly layer (the composite particle not having the protective layer). These points are on the surfaces of the metal-based particles, which surfaces form an interface with the protective layer. Then, a straight line is drawn to be shortest from each of the 10 points to an outer surface of the protective layer, and the average value of the lengths of the 10 straight lines thus obtained is obtained as the thickness of the protective layer.

In the composite particle according to one preferred embodiment, the protective layer covers the entire surface of the particle formed of the core particle and the metal-based particle assembly layer (the composite particle not having the protective layer). In this embodiment, the outer surface of the protective layer may have recesses and projections following the surface recesses and projections of the composite particle not having the protective layer (recesses and projections based on the metal-based particles), or the outer surface of the protective layer may be smooth without recesses and projections. Figure 3 shows an example in which the protective layer has surface recesses and projections. When the composite particle is applied to a sensor element, the protective layer preferably has surface recesses and projections, in view of increasing the amount of the analyte bound to the surface of the composite particle.

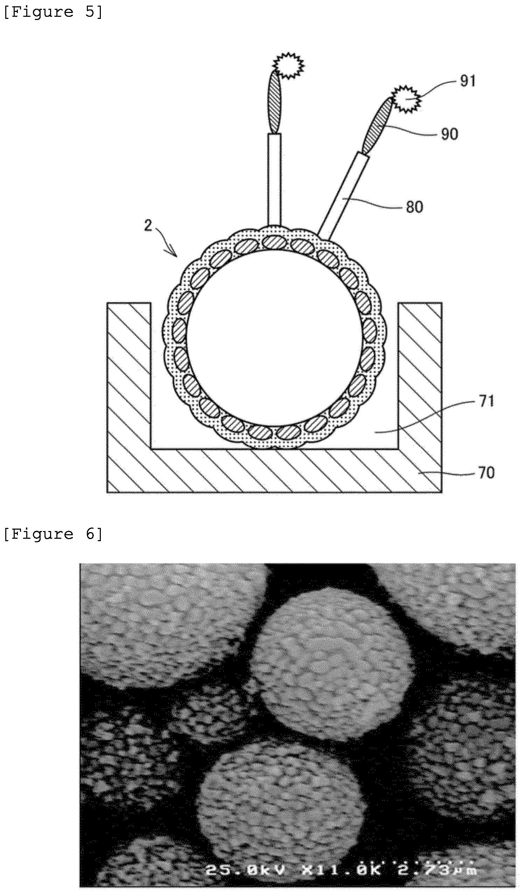

When the protective layer covers the entire surface of the composite particle not having the protective layer, the protective layer may be formed with a relatively uniform thickness over the entire surface or may be formed with a nonuniform thickness, such as thicker or thinner in local regions. When the composite particle is used as an emission enhancing element in a sensor element for capturing an analyte labeled with a luminescent substance onto the protective layer and analyzing the analyte, the protective layer is preferably formed with a uniform or relatively uniform thickness over the entire surface, in view of minimizing variations in the distance from the metal-based particle assembly layer to the luminescent substance depending on the position of the composite particle surface where the analyte labeled with the luminescent substance is captured. Moreover, as shown in Figure 5 described below, when the composite particle is used by being contained in a depression in a substrate, the composite particle in which the protective layer is formed with a nonuniform thickness may not be contained in the depression, which may lead to a detection failure in the composite particle.

On the other hand, when the composite particle having the protective layer has the shape of a true sphere or a shape close to a true sphere, for example, the composite particle can, for example, be stored in or passed through a substrate or filter fabricated on a microscale and envisaged to store the composite particle or allow the composite particle to pass therethrough, as envisaged. If the thickness of the protective layer is nonuniform, the composite particle may not take the behavior as envisaged and cause a defect.

<Method For Producing Composite Particle>

The present invention provides a method for producing a composite particle. Referring to Figures 1 and 2, the composite particle comprises the core particle 10 and the metal-based particle assembly layer arranged on at least a portion of a surface of the core particle 10. The metal-based particle assembly layer is an assembly of the plurality of metal-based particles 20 supported on the core particle 10, and is a layer comprising the plurality of metal-based particles 20 arranged apart from each other. In the example shown in Figures 1 and 2, the metal-based particle assembly layer is arranged on the entire surface of the core particle 10. Reference is made to the description in the <Composite Particle> section above for the specific structure of the composite particle obtained by the method described herein, the materials of the core particle, the metal-based particle assembly layer and the metal-based particles, and the like.

The method for producing a composite particle according to the present invention is suitable for use as a method for producing the composite particle described in the <Composite Particle> section above.

The method for producing a composite particle comprises the step of growing the plurality of metal-based particles on the surface of the core particle (metal-based particle growth step). Examples of methods of growing the metal-based particles include a sputtering method, a vapor deposition method (such as vacuum deposition), and a plating method.

(1) Sputtering Method

The sputtering method may, for example, be a method in which the core particle or the group of core particles are placed on a substrate, for example, a glass substrate, and the core particle or the group of core particles placed on the substrate are subjected to a sputtering process. The sputtering process can be performed, for example, by electrostatically adsorbing the core particle or the group of core particles on a substrate, and introducing the substrate into a sputtering apparatus.

It is noted that even when the sputtering process is performed by electrostatically adsorbing the core particle or the group of core particles on a substrate, and introducing the substrate into a sputtering apparatus, the metal-based particles can be grown on the surface of the core particle in contact with the substrate, by performing film deposition under the conditions as shown below, for example. This is because atoms of a metal-based material are sputtered at high pressure and low rate, which can sufficiently reduce the mean free path of the ejected atoms of the metal-based material (to, for example, 3 mm or less), and allows the atoms of the metal-based material to easily turn around the surface of the core particle. Moreover, if the amount of the core particle that is electrostatically adsorbed is adjusted to be relatively small so that the core particle can move on the substrate during film deposition, the atoms of the metal-based material can more easily turn around the surface of the core particle.

In the sputtering method, it is preferred to grow the metal-based particles at an extremely slow rate on the heated core particle or group of core particles, in view of forming the metal-based particle assembly layer with the preferred average particle diameter, average height, average interparticle distance and standard deviation thereof described above in a satisfactorily controlled manner.

The metal-based particles are grown on the surface of the core particle preferably at an average height growth rate of less than 1 nm/min, more preferably 0.5 nm/min or less, still more preferably 0.3 nm/min or less. As used herein, the average height growth rate can also be referred to as an average deposition rate or an average thickness growth rate of the metal-based particles, and is defined by the following expression:

average height of the metal-based particles/metal-based particle growth time

The "average height of the metal-based particles" is defined as set forth above.

The metal-based particle growth time refers to the time from the start to the end of the growth of the metal-based particles, specifically, the time during which the metal-based material is supplied. When the metal-based particles are grown by a sputtering method, the metal-based particle growth time is the sputtering time.

In the metal-based particle growth step, the temperature of the core particle, which may vary depending on the material of the metal-based particles, is preferably 80°C or more and 450°C or less, more preferably 100°C or more and 400°C or less, still more preferably 120°C or more and 400°C or less, and even more preferably 300°C or therearound (about 300°C $\pm$ 10°C) .

By adjusting the average height growth rate, the temperature of the core particle and/or the metal-based particle growth time, for example, the average particle diameter, the average height, the aspect ratio, the average interparticle distance and the standard deviation thereof of the plurality of metal-based particles grown on the core particle can be controlled.

While the pressure (pressure in the apparatus chamber) at which the metal-based particles are grown is not limited to a particular pressure as long as it allows the particles to be grown, the pressure is usually lower than atmospheric pressure. While the lower limit of the pressure is not limited to a particular value, it is preferably 0.5 Pa or more, more preferably 2 Pa or more, and still more preferably 10 Pa or more, because these pressures facilitate adjusting the average height growth rate within the above-mentioned range.

The sputtering may be performed in any manner, and may be performed using, for example, an ion gun, or a direct current argon ion sputtering method in which argon ions generated by plasma discharge are accelerated in an electric field and directed to a target. Other conditions such as current value, voltage value, and substrate-to-target distance in the sputtering method are adjusted appropriately to allow the metal-based particles to be grown at an average height growth rate of less than 1 nm/min. Alternatively, a method of directly depositing a film without adhering the core particle to the substrate may be used, and a film may be deposited while stirring a powder of the core particle continuously or as appropriate. For example, a method may be used in which a film is deposited while stirring the powder of the core particle as described above using sputtering (powder sputtering), vapor deposition (powder vapor deposition), CVD (powder CVD), or ALD.

In view of forming the metal-based particle assembly layer with the preferred average particle diameter, average height, average interparticle distance and standard deviation thereof described above in a satisfactorily controlled manner, it is preferred to set, in the metal-based particle growth step, the average height growth rate to less than 1 nm/min, and additionally, set the average particle diameter growth rate to less than 5 nm. Usually, the average particle diameter growth rate is less than 5 nm when the average height growth rate is less than 1 nm/min. The average particle diameter growth rate is more preferably 1 nm/min or less. The average particle diameter growth rate is defined by the following expression:

average particle diameter of the metal-based particles/metal-based particle growth time

The "average particle diameter of the metal-based particles" and the "metal-based particle growth time" are defined as set forth above.

To produce the metal-based particle assembly layer with the preferred average particle diameter, average height, average interparticle distance and standard deviation thereof described above, it is preferred to appropriately adjust the metal-based particle growth time in the metal-based particle growth step while considering the above-described preferred production conditions.

(2) Plating Method

The plating method includes, as the metal-based particle growth step, the step of forming the metal-based particle assembly layer on the surface of the core particle by immersing the core particle in a plating solution containing a cation of a metal constituting the metal-based particles (hereinafter also referred to as the "metal cation") and reducing the cation.

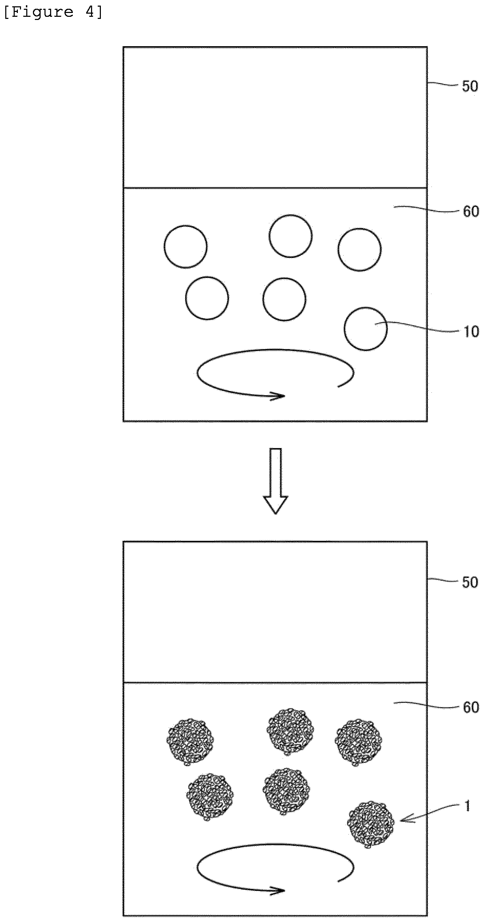

In this step, the core particle is immersed in a plating solution containing the metal cation constituting the metal-based particles, and reduction of the metal cation is performed. This step can be performed, for example, as shown in Figure 4, by adding the core particle 10 with stirring to a plating solution 60 containing the metal cation contained in a bath 50. When the core particle 10 is immersed in the plating solution 60 containing the metal cation, the metal (zero-valent) produced by reduction of the metal cation starts to deposit on the surface of the core particle 10. With passage of the reaction time, the deposited metal grows into particles to form an assembly of the metal-based particles (the metal-based particle assembly layer). The metal-based particle assembly layer is preferably formed on the entire surface of the core particle 10.

In one embodiment, the plating solution 60 can be prepared by mixing a metal cation solution containing the metal cation and a reducing agent solution containing a reducing agent. The metal cation solution containing the metal cation usually contains the metal cation and a solvent. The plating solution 60 may contain two or more types of metal cations.

The solvent contained in the metal cation solution is preferably a solvent capable of dissolving a corresponding metal salt contained as a metal cation source in the metal cation solution, for example, water. The metal cation solution may contain two or more solvents. The solvent may include, for example, water and an organic solvent (for example, an alcohol) miscible with water.

The metal cation concentration in the plating solution 60 is preferably 0.0008 mol/L or more, more preferably 0.002 mol/L or more, still more preferably 0.0035 mol/L or more, even more preferably 0.006 mol/L or more, and particularly preferably 0.01 mol/L or more. When the metal cation concentration falls in the above-mentioned range, the metal-based particles can be grown at a moderate rate, which facilitates forming the metal-based particle assembly layer with a preferred shape (average particle diameter, average height, average interparticle distance and standard deviation thereof) in a satisfactorily controlled manner. Moreover, when the metal cation concentration falls in the above-mentioned range, a sufficient amount of the metal-based particles can be easily formed on the entire surface of each core particle 10. To adjust the growth rate of the metal-based particles to a moderate rate, the metal cation concentration in the plating solution 60 is preferably 0.4 mol/L or less, more preferably 0.3 mol/L or less, still more preferably 0.15 mol/L or less, and even more preferably 0.05 mol/L or less.

In the metal-based particle growth step, a $V_S/V_L$ ratio of volume $V_S$ [cm$^3$] of the core particle 10 to volume $V_L$ [cm$^3$] of the plating solution 60 is preferably 0.03 or less. This facilitates forming the metal-based particle assembly layer containing the plurality of metal-based particles arranged apart from each other on the surface of the core particle 10. If the ratio is above 0.03, a continuous metal film is likely to be formed on the core particle 10, and it will be difficult to form the metal-based particle assembly layer in some cases. In view of facilitating forming the metal-based particle assembly layer, and facilitating forming the metal-based particle assembly layer with a preferred shape in a satisfactorily controlled manner, the ratio is more preferably 0.020 or less, still more preferably 0.010 or less, even more preferably 0.005 or less, particularly preferably 0.003 or less, and most preferably 0.002 or less. In view of mass productivity and the like of the composite particle, the ratio is usually 0.00001 or more, and may be 0.0001 or more, preferably 0.0005 or more, and more preferably above 0.0005. The volume Vs of the core particle 10 can be obtained by dividing the total particle weight by the particle density.

The plating solution 60 preferably contains the metal cation as well as a reducing agent capable of reducing the metal cation to a zero-valent metal. When the plating solution 60 contains a reducing agent, the reducing agent is preferably added into the plating solution 60 immediately before the core particle 10 is immersed in the plating solution 60. For example, as described above, the plating solution 60 containing the metal cation and the reducing agent can be prepared by mixing a metal cation solution containing the metal cation and a reducing agent solution containing a reducing agent. The reducing agent solution is a solution containing a reducing agent and a solvent, preferably a solution in which the reducing agent is dissolved in the solvent. The solvent contained in the reducing agent solution is preferably a solvent capable of dissolving the reducing agent, for example, water. The reducing agent solution may contain two or more solvents. The solvent may include, for example, water and an organic solvent (for example, an alcohol) miscible with water.

The reducing agent is preferably a reducing agent with a low reducing power, with a standard redox potential of preferably -0.5 V or more, more preferably -0.45 V or more. As used herein, the standard redox potential refers to the value obtained when using a standard hydrogen electrode as the cathode at pH 7 and 25°C. When the standard redox potential of the reducing agent used falls in the above-mentioned range, the metal-based particles can be grown at a moderate rate, which facilitates forming the metal-based particle assembly layer. The above-mentioned range of the standard redox potential is also advantageous in facilitating forming the metal-based particle assembly layer with a preferred shape in a satisfactorily controlled manner. If the reducing power of the reducing agent is excessively high, the growth rate of the metal-based particles will be excessively high, and a continuous metal film is likely to be formed.

Examples of reducing agents with a standard redox potential of -0.5 V or more include glucose and ascorbic acid. The plating solution 60 may contain two or more reducing agents.

The concentration of the reducing agent in the plating solution 60 is preferably 1 mol/L or less, more preferably 0.8 mol/L or less, still more preferably 0.4 mol/L or less, even more preferably 0.3 mol/L or less, and particularly preferably 0.25 mol/L or less. When the concentration of the reducing agent falls in the above-mentioned range, the metal-based particles can be grown at a moderate rate, which facilitates forming the metal-based particle assembly layer with a preferred shape. To adjust the growth rate of the metal-based particles to a moderate rate, the concentration of the reducing agent in the plating solution 60 is preferably 0.000001 mol/L or more, more preferably 0.000005 mol/L or more, still more preferably 0.000008 mol/L or more, even more preferably 0.00001 mol/L or more, and particularly preferably 0.0008 mol/L or more.

The ratio in percent of the concentration of the reducing agent in the plating solution 60 to the saturation concentration of the reducing agent in the plating solution 60 is preferably 8% or less, more preferably 4% or less, still more preferably 2% or less, even more preferably 1% or less, and particularly preferably 0.8% or less. When the ratio falls in the above-mentioned range, the metal-based particles can be grown at a moderate rate, which facilitates forming the metal-based particle assembly layer with a preferred shape in a satisfactorily controlled manner. To adjust the growth rate of the metal-based particles to a moderate rate, the ratio is preferably 0.0005% or more, more preferably 0.001% or more, still more preferably 0.003% or more, even more preferably 0.005% or more, and particularly preferably 0.01% or more. If the ratio is excessively high, it will be difficult to control the shape of the metal-based particle assembly layer, and a continuous metal film is likely to be formed.

The saturation concentration of the reducing agent in the plating solution 60 is the saturation concentration when the reducing agent is dissolved in the solvent (not containing the metal cation) contained in the plating solution 60 at the temperature at which the treatment of immersing the core particle 10 in the plating solution 60 is performed.

The plating solution 60 may contain one or more complexing agents that bind to the metal cation to form a complex ion and stabilize the metal cation, and one or more other additives. Examples of such complexing agents include amine-based complexing agents, such as ethylenediamine, ethylenediaminetetraacetic acid, tetrasodium ethylenediaminetetraacetate, and triethylenetetraminehexaacetic acid, ammonia, nitrotriacetic acid, sodium thiosulfate, succinic acid salts, succinimide, and citric acid salts or iodide salts. In view of adjusting the growth rate of the metal-based particles to a moderate rate, the complexing agent is preferably an amine-based complexing agent or ammonia, more preferably an amine-based complexing agent, and still more preferably ethylenediamine or the like. When preparing the plating solution 60 by mixing a metal cation solution and a reducing agent solution, the complexing agent may be previously added to the metal cation solution.

The treatment for forming the metal-based particle assembly layer comprising the plurality of metal-based particles 20 on the core particle 10 by immersing the core particle 10 in the plating solution 60 can basically be performed in the same manner as electroless plating (chemical plating) using a plating bath. The temperature at which the core particle 10 is immersed in the plating solution 60 is not limited to a particular temperature, and is, for example, 10°C or more and 100°C or less, preferably 15°C or more and 60°C or less, and more preferably 20°C or more and 40°C or less.

In view of facilitating forming the metal-based particle assembly layer and not a continuous metal film, and facilitating forming the metal-based particle assembly layer with a preferred shape in a satisfactorily controlled manner, it is preferred to adjust the growth rate of the metal-based particles to a moderate rate. Specifically, in the metal-based particle growth step, the average height growth rate of the metal-based particles at 28 minutes from the start of deposition of the metal onto the surface of the core particle 10 is preferably 6 nm/min or less, more preferably 5 nm/min or less, still more preferably 4 nm/min or less, and even more preferably 3.5 nm/min or less. When the ratio $V_S/V_L$ falls in the above-described range, the average height growth rate at 28 minutes from the start of deposition of the metal can be easily controlled in the above-mentioned range. In view of mass productivity and the like of the composite particle, the average height growth rate is usually 1 nm/min or more, and preferably 2 nm/min or more.

The average height growth rate of the metal-based particles at 28 minutes from the start of deposition of the metal onto the surface of the core particle 10 can be measured by, for example, previously performing, as a preliminary experiment, the same metal-based particle growth step except that the reaction is terminated (for example, filtered off) at 28 minutes, and measuring the average height growth rate from an average height obtained in the preliminary experiment.

The phrase "start of deposition of the metal onto the surface of the core particle 10" means the start of immersion of the core particle 10 when the metal-based particle growth step is initiated by immersing the core particle 10 in the plating solution 60 containing the metal cation and a reducing agent, or means the time of addition of reducing agent when the core particle 10 is immersed in a metal cation solution containing the metal cation, and then a reducing agent (for example, a reducing agent solution) is added.

In view of facilitating forming the metal-based particle assembly layer and not a continuous metal film, and facilitating forming the metal-based particle assembly layer with a preferred shape in a satisfactorily controlled manner, the metal-based particle growth step preferably satisfies the following expression (1):

$$10 \times 10^{-7} \leq X \leq 600 \times 10^{-7} \quad (1)$$

When the ratio $V_S/V_L$ falls in the above-described range, the value of X can be easily controlled in the above-mentioned range. The value of X is more preferably $30 \times 10^{-7}$ or more and $450 \times 10^{-7}$ or less, and still more preferably $40 \times 10^{-7}$ or more and $300 \times 10^{-7}$ or less.

When the core particle 10 is spherical, X is represented by the following expression:

[Expression 1]

$$X = (\frac{3aVD}{4\pi BC} + R^3)^{\frac{1}{3}} - R$$

wherein a represents the metal cation concentration (mol/L) in the plating solution 60; V represents the volume (L) of the plating solution 60; D represents the atomic weight of the metal (zero-valent) produced by reduction of the metal cation; B represents the particle number (particles) of the core particle 10; C represents the specific gravity ($g/cm^3$) of the metal (zero-valent) produced by reduction of the metal cation; and R represents the radius (cm) of the core particle 10.

When the core particle 10 has an irregular shape, X is represented by the following expression:

[Expression 2]

$$X = \frac{aVD}{SC}$$

wherein a, V, D, and C represent the same meanings as set forth above; and S represents a sum total of surface areas of the core particle 10.

The particle number B of the core particle 10 can be obtained from the total weight $m_p$ of the particle and the density $V_p$ of the particle, according to the following expression:

[Expression 3]

$$B = \frac{3V_p}{4\pi R^3 m_p}$$

wherein R represents the same meaning as set forth above. The radius R (cm) of the core particle 10 is half the average particle diameter of the core particles 10 obtained as described above.

The sum total S of surface areas of the core particle 10 can be obtained by measuring the specific surface area by the BET method, and multiplying the result by the total weight $m_p$ of the particle.

The phrase "core particle 10 is spherical" means that the core particle 10 has the following shape: A SEM image is obtained in the same manner as above, and, in all 10 particles adopted, all five tangential diameters adopted are ±30% of the average tangential diameter (i.e., the average particle diameter of the core particles 10 × 70% or more and 130% or less). "Amorphous" refers to shapes that are not spherical.

The plating method also allows the metal-based particle assembly layer comprising the plurality of metal-based particles 20 to be formed on the surface of the core particle 10 in a satisfactorily controlled manner, and further allows the metal-based particle assembly layer with a preferred shape to be formed on the surface of the core particle 10 in a satisfactorily controlled manner. Moreover, the plating method also facilitates forming the metal-based particle assembly layer over the entire surface of the core particle 10. Furthermore, the plating method can improve mass productivity of the composite particle production.

The time of the metal-based particle growth step, i.e., the time in which the core particle 10 is immersed in the plating solution 60 to grow the metal-based particles 20, is preferably appropriately controlled. If the time is excessively long, the metal-based particle assembly layer comprising the plurality of metal-based particles 20 will not be formed, and instead, a continuous metal film will be formed. The longer the time, the larger the average particle diameter and average height of the metal-based particles tends to be, and the smaller the average interparticle distance tends to be. The appropriate time of the metal-based particle growth step is preferably previously known from a preliminary experiment before the metal-based particle growth step is actually performed.

(3) Step of Performing Heat Treatment

After the metal-based particle growth step, the step of subjecting the core particle having the metal-based particles supported thereon to a heat treatment may be further carried out. The heat treatment can serve to, for example, equalize the amount of the supported metal-based particles and the distribution of the presence of the metal-based particles, evaporate excess metal-based particle material, and adjust the shape of the metal-based particles (increase the particle diameter and equalize the height).

The temperature of the heat treatment, which may vary depending on the material of the metal-based particles, is preferably 150°C or more and 550°C or less, more preferably 200°C or more and 500°C or less, still more preferably 250°C or more and 450°C or less, even more preferably 300°C or more and 450°C or less, and particularly preferably 400°C or thereabout (about 400°C ± 10°C).

(4) Step of Forming Protective Layer

When producing a composite particle further comprising a protective layer, the step of forming a protective layer is carried out after the metal-based particle growth step. The protective layer is formed to cover at least surfaces of the plurality of metal-based particles on the surface of the core particle. Reference is made to the description in the <Composite Particle> section above for the structure, material, and the like of the protective layer.

Examples of methods of forming the protective layer include a method in which a liquid containing a protective layer material is spin-coated onto the particle formed of the core particle and the metal-based particle assembly layer (the composite particle not having the protective layer) placed on a substrate. The composite particle not having the protective layer may be electrostatically adsorbed on a substrate.

Examples of other methods of forming the protective layer include dry film deposition methods, such as a sputtering method (which may be powder sputtering), a vapor deposition method (which may be powder vapor deposition), and CVD (which may be powder CVD); and a wet method, such as a method in which the composite particle not having the protective layer is dispersed in a liquid containing a material for forming the protective layer to form the protective layer on the surface of the composite particle.

One suitable example of the wet method is a sol-gel method. The sol-gel method is a method that starts from a solution containing a metal alkoxide or the like as a raw material, and involves the hydrolysis reaction and polymerization reaction, leading to a corresponding metal oxide. One example of the sol-gel method is the Stober method ("o" in "Stober" means the umlaut of "o"; the same applies hereinafter). With this method, silica ($SiO_2$) can be produced by using tetraethyl orthosilicate as a raw material, and performing the reaction in an ethanol solvent in the presence of water and ammonia as a catalyst. For the Stober method, reference may be made to W. Stober and A. Fink, Journal of Colloid and Interface Science 26(1), 62-69 (1968).

With the Stober method, a $SiO_2$ layer can be formed as a protective layer on the composite particle formed of the core particle and the metal-based particle assembly layer. Specifically, a $SiO_2$ layer can be formed on the composite particle, by performing the above-mentioned reaction of tetraethyl orthosilicate or the like as a raw material in a solution containing a solvent such as an alcohol, water, and ammonia as a catalyst, in the presence of the composite particle formed of the core particle and the metal-based particle assembly layer.

While the temperature of the above-mentioned reaction may be selected as appropriate, it is, for example, 0°C or more and 100°C or less, and preferably 10°C or more and 60°C or less. During the reaction, the reaction solution may be subjected to an ultrasonic treatment, which facilitates forming a uniform coating with the protective layer.

The thickness of the protective layer depends on the amount of raw material (such as a metal alkoxide) relative to the composite particle formed of the core particle and the metal-based particle assembly layer in the reaction solution. A $V_G/V_{L2}$ ratio of volume $V_G$ [$cm^3$] of the raw material to volume $V_{L2}$ [$cm^3$] of the composite particle formed of the core particle and the metal-based particle assembly layer in the reaction solution is, for example, 15 or less, preferably 9 or less, and more preferably 6 or less. The ratio is usually 0.1 or more. Increasing the ratio tends to increase the thickness of the protective layer.

The material that can be used to form the protective layer formed of a metal oxide using the sol-gel method is not limited to tetraethyl orthosilicate, and may also be methyltrimethoxysilane, for example.

<Other Embodiments of Composite Particle>

A composite particle according to this embodiment comprises a core particle; a metal-based material layer arranged on at least a portion of a surface of the core particle; and a protective layer that covers a surface of the metal-based material layer. Reference is made to the description in the <Composite Particle> section above for the core particle and the protective layer in the composite particle according to this embodiment. The metal-based material layer preferably covers an entire surface of the core particle. The protective layer preferably covers an entire surface of the metal-based material layer.

The metal-based material layer of the composite particle according to this embodiment may be, for example, a metal-based particle assembly layer comprising a plurality of metal-based particles arranged apart from each other, as stated in the <Composite Particle> section above, or a continuous film composed of a metal-based material. Examples of the metal-based material include those exemplified as the metal-based material constituting the metal-based particles. Reference is made to the description in the <Composite Particle> section above for the metal-based particle assembly layer.

The thickness of the metal-based material layer in the form of a continuous film is, for example, 5 nm or more and 500 nm or less, preferably 5 nm or more and 200 nm or less, and more preferably 5 nm or more and 50 nm or less.

The thickness of the metal-based material layer is measured according to the following method: The scanning electron microscope "JSM-5500" produced by JEOL Ltd. or its equivalent is used to obtain a cross-sectional image of the composite particle. In the obtained cross-sectional image, 10 points on an outer contour surface of the core particle are selected at random. Then, a straight line is drawn to be shortest from each of the 10 points to an outer surface of the metal-based material layer, and the average value of the lengths of the 10 straight lines thus obtained is obtained as the average thickness of the metal-based material layer.

The composite particle comprising the metal-based material layer in the form of a continuous film can be used as, for example, a light scattering particle. In this case, the composite particle is used by being contained in various members in which light scattering properties are required.

The composite particle according to this embodiment comprising the protective layer on the metal-based material layer can increase the stability (such as oxidation resistance) and environmental stability (for example, light resistance, humidity resistance, and heat resistance) of the composite particle (the core particle and/or the metal-based particles).

<Sensor Element>

The sensor element according to the present invention (hereinafter also simply referred to as the "sensor element") is a sensor element used for detecting an analyte, comprising a substrate; a composite particle arranged on the substrate; and a capturing portion that is arranged on the composite particle and has a capturing substance that specifically binds to the analyte.

The composite particle of the sensor element comprises a core particle and a metal-based particle assembly layer arranged on at least a portion of a surface of the core particle. The metal-based particle assembly layer is an assembly of a plurality of metal-based particles supported on the core particle, and is a layer comprising the plurality of metal-based particles arranged apart from each other. The composite particle can further comprise a protective layer that covers at least surfaces of the plurality of metal-based particles. The composite particle according to the present invention can be used as the composite particle. Reference is made to the description in the <Composite Particle> section above for the specific structure of the composite particle used in the sensor element, the materials of the core particle, the metal-based particle assembly layer and the metal-based particles, and the like.

Figure 5 is a cross-sectional view schematically showing one example of the sensor element. The sensor element shown in Figure 5 includes a substrate 70 having a depression 71 in a surface; the composite particle 2 according to the present invention arranged on the substrate 70, more specifically in the depression 71; and a capturing portion 80 that is arranged on the composite particle 2 and has a capturing substance that specifically binds to an analyte 90. While the capturing portion 80 is provided on the protective layer of the composite particle 2 in the example shown in Figure 5, the composite particle not having the protective layer may be used, and the capturing portion 80 may be provided on the core particle and/or on the metal-based particle assembly layer.

Figure 5 schematically shows the labeled analyte 90 being captured by the capturing portion 80. In Figure 5, reference numeral 91 designates the label bound to the analyte 90. Examples of the label include a luminescent substance.

The sensor element detects the analyte 90 as follows: The detection may be qualitative or quantitative, and refers to, for example, identification or quantification of the analyte 90. When excitation light is directed to the labeled analyte 90 specifically bound to the capturing substance on the composite particle 2, the label, which is a luminescent substance, is excited. Then, the metal-based particle assembly layer of the composite particle 2 resonates with the excited luminescent substance to achieve plasmonic emission enhancement. The analyte 90 can be detected qualitatively or quantitatively by detecting the emission from the excited luminescent substance using a detector. For example, the amount of the analyte 90 that is present can be measured qualitatively or quantitatively by measuring the emission intensity.

In the sensor element comprising the composite particle according to the present invention, the composite particle comprises the metal-based particle assembly layer and can thereby achieve plasmonic emission enhancement, thus leading to increased detection sensitivity and detection accuracy. For example, it may be conceivable to arrange the metal particle assembly layer on the bottom surface of the depression 71 of the substrate 70, and arrange a bead (corresponding to the core particle) not having the metal particle assembly layer in the depression 71 instead of the composite particle; however, in this case, if the bead having the capturing portion on the surface has a large particle diameter (for example, on the order of micrometers), the labeled analyte 90 and the metal particle assembly layer may be too far from each other to obtain the plasmonic emission enhancing effect. In contrast, when the composite particle comprising the metal-based particle assembly layer is used as a bead arranged in the depression 71, the distance between the labeled analyte 90 and the metal particle assembly layer is reduced, such that the plasmonic emission enhancing effect can be obtained.

Moreover, the composite particle in which the metal-based particle assembly layer is arranged on the surface of the core particle and the composite particle in which the protective layer is further arranged on the surface of the particle have surface recesses and projections or may be formed to have surface recesses and projections. The composite particle with surface recesses and projections has a larger surface area. Thus, when the composite particle is used as the bead, a larger amount of the capturing substance and hence the analyte 90 can be bound to the surface of the composite particle. This also contributes to improving the detection sensitivity and detection accuracy.

The analyte 90 is a substance on which a qualitative or quantitative detection is to be performed, and is a substance that specifically binds to the capturing substance. Examples of the analyte 90 include, without particular limitation, nucleosides, nucleotides, nucleic acids, proteins, sugars, glycoproteins, lectin, viruses, cells, antibodies, and exosomes. A sensor element for which the analyte 90 is a biological material or a biologically relevant material is also referred to as a biosensor element.

The nucleic acids mean polymers of phosphates of nucleosides (nucleotide chains) in which purine or pyrimidine bases and sugar are glycosidically linked, and include oligonucleotides including probe DNA, polynucleotides, DNA formed by polymerization of purine nucleotides and pyrimidine nucleotides (full length or fragments thereof), RNA, and polyamide nucleotide derivatives (PNA). The nucleosides are compounds in which bases and sugar are glycosidically linked, and the nucleotides are compounds in which phosphoric acid is linked to the nucleosides; the nucleosides and nucleotides are compounds containing bases.

The term "specifically bind" broadly means chemical bonding including non-covalent bonding, covalent bonding, and hydrogen bonding between substances, for example, interactions between protein molecules and electrostatic interactions between molecules.

The analyte 90 captured can be detected by pre-labeling the analyte 90 with the label 91, which is a luminescent substance, and detecting emission from the label 91. The label 91 may be a labeling substance that specifically binds to a complex obtained by the specific binding between the capturing substance and the analyte 90. The luminescent substance is a substance that emits light upon injection of excitation energy, such as excitation light. The principle of emission of light by the luminescent substance is not limited to a particular principle, and includes fluorescence, phosphorescence, and chemiluminescence. The luminescent substance may be a conventionally known luminescent substance.

The capturing substance constituting the capturing portion 80 is a substance that functions to capture the analyte 90 by specifically binding thereto. The capturing substance is, for example, fixed on the surface of the composite particle. The capturing substance is, for example, a substance having binding active groups that can specifically bind to the analyte 90. Examples of such binding active groups include carboxyl and hydroxyl groups that can electrostatically interact with the analyte 90. Examples of the capturing substance include, without particular limitation, nucleosides, nucleotides, nucleic acids, proteins, sugars, and glycoproteins.

Examples of materials of the substrate 70 of the sensor element include silicon, quartz, synthetic quartz, glass, and thermoplastic resins. The composite particle is not necessarily limited to being arranged in the depression 71 of the substrate 70, and may be arranged in such a way that it can be fixed or contained in the substrate 70. The substrate 70 can have one or more depressions 71. Examples of the substrate 70 include a flow cell and a microarray.

The sensor element according to the present invention includes the composite particle, which has a broad range of the effect of localized plasmon resonance, and thus, can enhance the emission from the label 91 even if the distance from the metal-based particle assembly layer of the composite particle to the label 91 is long, i.e., above 10 nm.

For example, DNA that may be the analyte 90 may have a size of about several nm to several ten nm, and may be 5 nm to 15 nm. For example, a virus may have a size of about several ten nm to several hundred nm, and may be 30 nm to 120 nm. When each of these substances is the analyte 90, the distance between the label 91 bound thereto and the metal-based particle assembly layer may be several ten nm to several hundred nm, for example. The sensor element according to the present invention can enhance the emission from the label 91 even in this case.

The distance from the metal-based particle assembly layer to the label 91 may be 15 nm or more, further 25 nm or more, and still further equal to and more than that. The distance is preferably 200 nm or less, more preferably 170 nm or less, and still more preferably 150 nm or less, in view of the sensitivity of the sensor element.

The maximum wavelength of the plasmon peak of the metal-based particle assembly layer preferably coincides with or is close to the emission wavelength of the label 91. This can more effectively increase the emission enhancing effect of plasmon resonance. The maximum wavelength of the plasmon peak of the metal-based particle assembly layer can be controlled by adjusting the metal species, the average particle diameter, the average height, the aspect ratio, and the average interparticle distance and/or the standard deviation of the average interparticle distance of the metal-based particles constituting the metal-based particle assembly layer.

A sensor apparatus comprising the sensor element according to the present invention usually includes, in addition to the sensor element, a light source that emits excitation light for exciting the label 91 and a detector that detects emission from the label 91. The light source and the detector may be arranged either on different sides relative to the substrate 70 or on the same side. In one embodiment, the light source is arranged above the substrate 70 (in Figure 5, above or laterally of the labeled analyte 90), while the detector is arranged below (on the rear side of) the substrate 70, i.e., opposite to the light source relative to the substrate 70, or on the same side as the light source. When the detector is arranged below (on the rear side of) the substrate 70, the substrate 70 preferably has light-transmitting properties, and more preferably is optically transparent. The substrate 70 with light-transmitting properties preferably has a light transmittance of 80% or more, more preferably a light transmittance of 90% or more, with respect to the light that is to be transmitted therethrough.

If the excitation light may be mixed with the emission during detection of the emission, it is preferred to allow the emission to enter the detector through a wavelength cut filter that cuts off light at the wavelength of the excitation light.

Examples of the sensor apparatus include a DNA sequencer, a DNA microarray, a virus sensor, an ion sensor, a plate reader (such as a protein chip, a sugar chain chip, or a lectin chip), a microspectrometer, and a biosensor such as a glucose sensor.

It is noted that when the composite particle according to the present invention is used for sensor applications, the composite particle may be used without being fixed to a substrate. Examples of such methods of use include use in flow cytometry, and a method in which the composite particle is dispersed in a liquid medium, and the labeled analyte 90 is bound to the dispersed composite particle to perform analysis.

Examples

The present invention will be described in more detail with reference to examples, although the present invention is not limited thereto.

<Example 1>

(1) Step of Growing Metal-Based Particles

Silica powder ("X-52-7042" produced by Shin-Etsu Silicone Co., Ltd.; a group of spherical particles with an average particle diameter of 4 $\mu$m) as core particles were electrostatically adsorbed on a glass substrate. The glass substrate was introduced into a direct-current magnetron sputtering apparatus so that the silica powder adsorbed on the glass substrate faced vertically downward.

The direct-current magnetron sputtering apparatus was used under the following conditions to grow silver particles extremely slowly on the surface of the heated silica powder to form a metal-based particle assembly layer over the entire surface of the silica powder to produce composite particles.

gas used: argon;
pressure in chamber (sputtering-gas pressure): 10 Pa;
substrate-to-target distance: 100 mm;
sputtering power: 4 W;
average particle diameter growth rate (average particle diameter/sputtering time) of the silver particles: 0.87 nm/min;

average height growth rate (= average deposition rate = average height/sputtering time) of the silver particles: 0.16 nm/min;

silica powder heating temperature: 300°C

sputtering time: 360 min

(2) Step of Performing Heat Treatment

The composite particles obtained in step (1) were heat-treated at 400°C in air for 1 minute.

Figure 6 shows a SEM image (at a scale of 11000 times) of the composite particles obtained (after the heat treatment step). The scanning electron microscope "JSM-5500" produced by JEOL Ltd. was used herein to obtain SEM images, including this SEM image.

From the SEM image, in the composite particles of Example 1, the silver particles constituting the metal-based particle assembly layer covering the core particles were determined to have an average particle diameter of 312.4 nm, an average interparticle distance of 30.7 nm, and a standard deviation of the average interparticle distance of 18.6 nm, based on the above-described definitions.

The average height of the silver particles was determined as 57.9 nm based on the results of AFM imaging using "VN-8010" produced by KEYENCE CORPORATION. Thus, the aspect ratio (average particle diameter/average height) of the silver particles was calculated as 5.4.

It is noted that the average particle diameter (4 $\mu$m) of the silica powder used as the core particles was determined according to the method of measuring the average particle diameter of a group of core particles described above.

<Example 2>

The composite particles obtained in Example 1 were electrostatically adsorbed on a glass substrate. Then, a protective layer was layered on the composite particles by spin-coating a spin-on-glass (SOG) solution onto the glass substrate having the composite particles adsorbed thereon to produce composite particles including the protective layer.

The SOG solution was a solution obtained by diluting the organic SOG material "OCD T-7 5500T" produced by Tokyo Ohka Kogyo Co., Ltd. with ethanol.

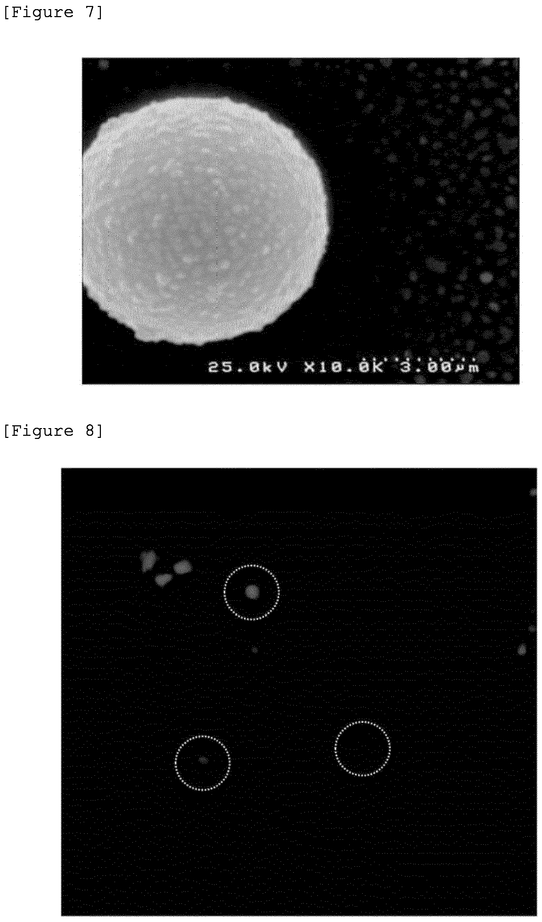

Figure 7 shows a SEM image (at a scale of 10000 times) of the obtained composite particles including a protective layer. It could be observed from the SEM image that the protective layer was formed over the entire surfaces of the composite particles obtained in Example 1. It was also observed that the protective layer was formed not only on the surfaces of the silver particles of the metal-based particle assembly layer, but also in gaps between the silver particles (where the core particle was exposed).

<Comparative Example 1>

The core particles (silica powder) used in Example 1 were used as the particles of Comparative Example 1.

(Evaluation of Fluorescence Intensity Enhancing Effect)

The composite particles obtained in Example 1 were electrostatically adsorbed on a glass substrate. Then, rhodamine B solution was spin-coated onto the glass substrate having the composite particles adsorbed thereon at 2000 rpm for 100 seconds to make the fluorescent dye, which is rhodamine B, supported on the surfaces of the composite particles. The resulting composite particles are designated as measurement sample 1. 0.15 mM rhodamine B solution of "Rhodamine B" produced by Exciton dissolved in ethanol was used as a Rhodamine B solution.

Separately, the fluorescent dye, which is rhodamine B, was supported on the surfaces of the particles of Comparative Example 1, using the same method as described above. The resulting composite particles are designated as measurement sample 2.

For the measurement samples 1 and 2, a fluorescence microscope ("FV1000" produced by Olympus Corporation) was used to measure fluorescence intensities emitted from rhodamine B supported on the composite particles (measurement sample 1) and rhodamine B supported on the silica particles (measurement sample 2) when irradiated with laser light.

The conditions for measuring the fluorescence intensities were as follows:

laser excitation wavelength: 559 nm
detection condition: Cy5
objective lens: 40 times

In the fluorescence image obtained by the measurement of the measurement sample 1, five composite particles not in contact with others were selected at random, the average value of the "INT" values was calculated, the background value was subtracted from this average value, and the resulting value was obtained as a fluorescence intensity 1. The fluorescence intensity obtained by the measurement of the measurement sample 2 in the same manner was obtained as a fluorescence intensity 2. The composite particles not in contact with others (measurement sample 1) and the silica particles not in contact with others (measurement sample 2) were selected because, particularly when the silica particles, which are transparent particles, are in contact with each other, they retroreflect the excitation light and emission inside the particles to each another, which causes variations in fluorescence intensity.

The background value was obtained as follows: For an untreated glass substrate (the same substrate as used in the measurement samples 1 and 2), an image was obtained by measuring the fluorescence intensity under the same conditions as shown above. Five points were selected at random from this image, and the average value of the "INT" values was calculated and designated as the background value.

The fluorescence intensity 1 was about 5.1 times higher than the fluorescence intensity 2.

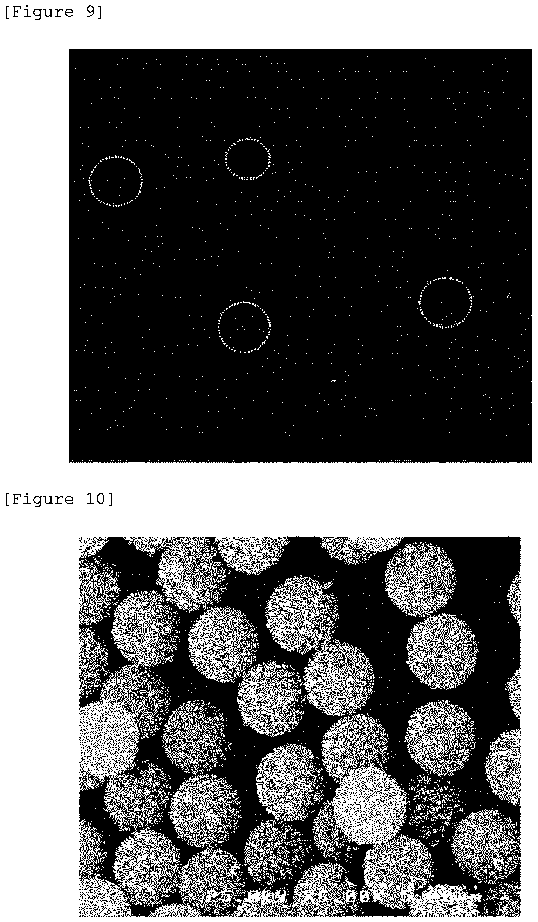

Figure 8 shows the image obtained by fluorescence intensity measurement for the measurement sample 1, and Figure 9 shows the image obtained by fluorescence intensity measurement for the measurement sample 2.

<Example 3>

0.25 mL of a 0.05 mol/L aqueous potassium hydroxide (KOH) solution was added dropwise to 50 mL of a 0.047 mol/L aqueous silver nitrate (AgNOs) solution, and then the mixture was stirred. Upon the addition of aqueous potassium hydroxide solution, the solution turned from clear and colorless to brown in color. To this solution, a 3.5 mol/L aqueous ethylenediamine ($NH_2CH_2CH_2NH_2$) solution was added dropwise in 50 $\mu$L portions with stirring, and the addition was stopped when the solution turned clear and colorless. The resulting solution is designated as a silver ion solution A.

16.6 mL of a 0.06 mol/L aqueous glucose solution and 8.4 mL of methanol were mixed to produce a reducing agent solution B. The silver ion solution A was added into a bath, and then 200 mg of silica powder ("Fine Sphere SK-30" produced by Nippon Electric Glass Co., Ltd.; a group of spherical particles with an average particle diameter of 3 $\mu$m) as core particles were added into the bath. Then, immediately after addition of the reducing agent solution B into the bath, the solution in the bath was stirred, thereby immersing the core particles in the plating solution containing the silver ion solution A and reducing agent solution B, which initiated growth of the metal-based particles formed of silver on the surfaces of the core particles. The preparation of the plating solution and the immersion of the core particles in the plating solution were performed in an environment at 25°C. The stirring was continued at 25°C. After 28 minutes from the start of immersion, the core particles having the metal-based particle assembly layer (composite particles) were filtered off, the filtrate was then washed with a 1:1 by volume mixture of acetone and ultrapure water, and then the supernatant was removed using a centrifuge and then dried at 80°C to produce composite particles.

<Example 4>

Composite particles were produced by performing the metal-based particle growth step as in Example 3, except that the concentration of glucose in the aqueous glucose solution used to prepare the reducing agent solution B was changed so that the concentration of the reducing agent and the ratio (in percent) of the concentration of the reducing agent to the saturation concentration were the values as shown in Table 1.

<Comparative Example 2>

The core particles (silica powder) used in Example 3 were used as the particles of Comparative Example 2. It is noted that the average particle diameter (3 $\mu$m) of the silica powder used as the substrates was determined according to the method of measuring the average particle diameter of a group of particles described above.

Table 1 shows, for Examples 3 and 4, the silver ion concentration in the plating solution, the type of reducing agent, the concentration of reducing agent, the ratio (in percent) of the concentration of reducing agent to the saturation concentration, the immersion time of the core particle in the plating solution, the $V_S/V_L$ ratio of volume $V_S$ [cm$^3$] of the core particle to volume $V_L$ [cm$^3$] of the plating solution, and the average height growth rate of the metal-based particles at 28 minutes from the start of deposition of the metal.

In both Examples 3 and 4 in which the silica powder in the form of spherical particles was used as the core particles, the value of X obtained based on the expression shown above was $121 \times 10^{-7}$.

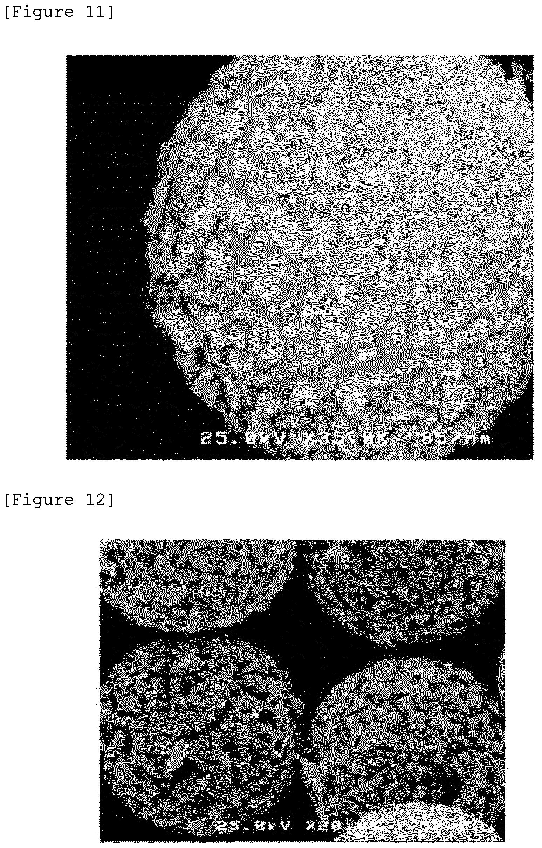

Figures 10 and 11 show SEM images (Figure 10: at a scale of 6000 times; Figure 11: at a scale of 35000 times) of the composite particles obtained in Example 3. Figure 12 shows a SEM image (at a scale of 20000 times) of the composite particles obtained in Example 4.

From the SEM image, the average particle diameter and average interparticle distance based on the above-described definitions of the silver particles constituting the metal-based particle assembly layer covering the core particles were determined. The average height based on the above-described definition of the silver particles was also determined, based on a STEM cross-sectional image (at a scale of 100000 times). The STEM cross-sectional image was obtained using the scanning transmission electron microscope "Helios G4 UX" produced by FEI Company. The aspect ratio of the silver particles was calculated from the average particle diameter and average height obtained. These results are shown in Table 1. It is noted that, for the composite particles of Example 4, a SEM image was obtained (Figure 12), but the average particle diameter and average interparticle distance were not obtained. As shown in Figure 12, although the metal-based particle assembly layer of the composite particles of Example 4 contained a plurality of metal-based particles arranged apart from each other, the composite particles of Example 4 included more regions where the metal-based particles were connected to form a continuous film, as compared to the composite particles of Example 3.

[Table 1]

| | | | | Examples | |
| --- | --- | --- | --- | --- | --- |
| | | | | 3 | 4 |
| Metal-Based Particle Growth Step | Plating Solution | Silver Ion Concentration | mol/L | 0.031 | 0.031 |
| | | Type of Reducing Agent | - | Glucose | Glucose |
| | | Concentration of Reducing Agent | mol/L | 0.0133 | 0.0421 |
| | | Ratio of Concentration of Reducing Agent to Saturation Concentration | % | 0.50 | 1.59 |
| | Immersion Time of Core Particle in Plating Solution | | min | 28 | 28 |
| | $V_S/V_L$ | | - | 0.0012 | 0.0121 |
| | Average Height Growth Rate | | nm/min | 3.2 | 4.1 |
| Physical Properties of Metal-Based Particle Assembly Layer | Average Particle Diameter | | nm | 304.6 | - |
| | Average Height | | nm | 89.7 | 114.0 |
| | Aspect Ratio | | | 3. 40 | - |
| | Average Interparticle Distance | | nm | ss. $\alpha$ | - |

(Evaluation of Fluorescence Intensity Enhancing Effect)

A spin-on-glass (SOG) solution was spin-coated onto a glass substrate at 3000 rpm, and then the composite particles obtained in Example 3 were placed on the SOG coating film. Then, the SOG coating film was dried at 200°C to fix the composite particles. The SOG solution was "OCD T-7 5500T" produced by TOKYO OHKA KOGYO CO., LTD, which is an organic SOG material.

Next, rhodamine B solution was spin-coated onto the surface having the composite particles fixed thereon at 2000 rpm for 100 seconds to make the fluorescent dye, which is rhodamine B, supported on the surfaces of the composite particles. The resulting composite particles are designated as measurement sample 3. 0.15 mM rhodamine B solution of "Rhodamine B" produced by Exciton dissolved in ethanol was used as a Rhodamine B solution.

Separately, the fluorescent dye, which is rhodamine B, was supported on the surfaces of the particles of Comparative Example 2, using the same method as described above. The resulting composite particles are designated as measurement sample 4.

For the measurement samples 3 and 4, a fluorescence microscope ("FV1000" produced by Olympus Corporation) was used to measure fluorescence intensities emitted from rhodamine B supported on the composite particles (measurement sample 3) and rhodamine B supported on the silica particles (measurement sample 4) when irradiated with laser light. The conditions for measuring the fluorescence intensities were the same as shown above.

In the fluorescence image obtained by the measurement of the measurement sample 3, 10 composite particles not in contact with others were selected at random, the average value of the "INT" values was calculated, the background value was subtracted from this average value, and the resulting value was obtained as a fluorescence intensity 3. The fluorescence intensity obtained by the measurement of the measurement sample 4 in the same manner was obtained as a fluorescence intensity 4. The composite particles not in contact with others (measurement sample 3) and the silica particles not in contact with others (measurement sample 4) were selected because, particularly when the silica particles, which are transparent particles, are in contact with each other, they retroreflect the excitation light and emission inside the particles to each another, which causes variations in fluorescence intensity.

The background value was obtained as follows: For an untreated glass substrate (the same substrate as used in the measurement samples 3 and 4), an image was obtained by measuring the fluorescence intensity under the same conditions as shown above. Ten points were selected at random from this image, and the average value of the "INT" values was calculated and designated as the background value.

The fluorescence intensity 3 was 19.7 times higher than the fluorescence intensity 4.

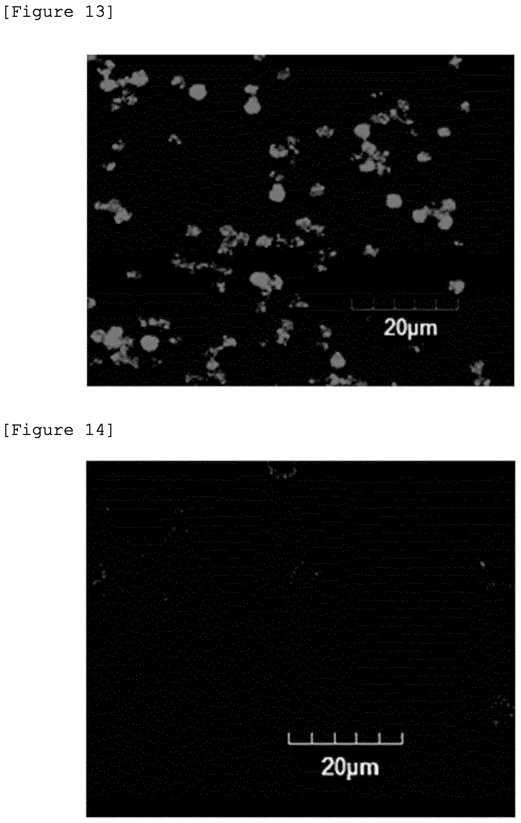

Figure 13 shows the image obtained by fluorescence intensity measurement for the measurement sample 3, and Figure 14 shows the image obtained by fluorescence intensity measurement for the measurement sample 4.

<Example 5>

A solution was obtained by dissolving 1188 μL of a 25 mass% aqueous ammonia (NH$_3$) solution in 5000 μL of ethanol. This solution was heated to 50°C, and then 24.8 mg of the composite particles obtained in Example 3 and 32.4 μL of tetraethyl orthosilicate were added with stirring at the same temperature to produce a reaction solution. The stirring of the reaction solution at 50°C was continued for 4 hours while subjecting the reaction solution to an ultrasonic treatment, and then the supernatant was removed using a centrifuge, and washed with water and dried at 80°C sequentially to produce composite particles in which a protective layer formed of SiO$_2$ was formed on the metal-based particle assembly layer. The $V_G/V_{L2}$ ratio of volume $V_G$ [cm$^3$] of tetraethyl orthosilicate to volume $V_{L2}$ [cm$^3$] of the composite particle formed of the core particle and the metal-based particle assembly layer in the reaction solution was 2.87.

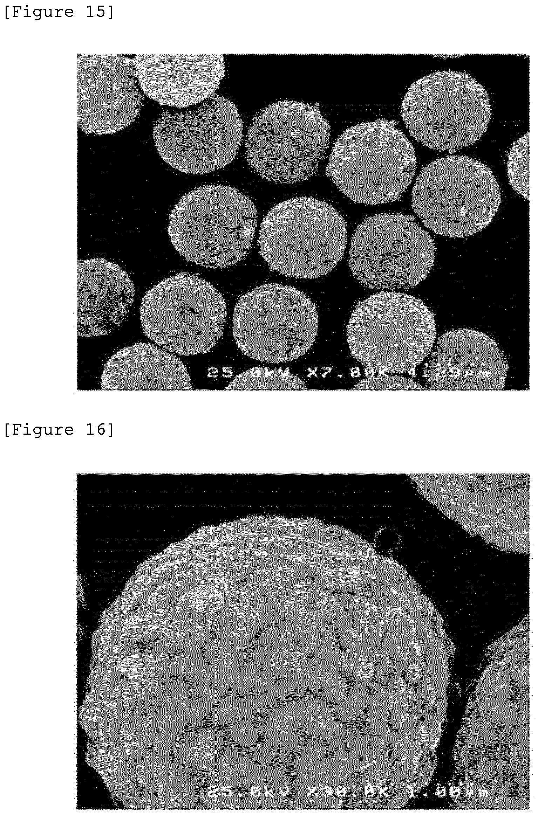

Figures 15 and 16 show SEM images (Figure 15: at a scale of 7000 times; Figure 16: at a scale of 30000 times) of the obtained composite particles including a protective layer. It could be observed from the SEM images that the protective layer was formed over the entire surfaces of the composite particles obtained in Example 3. It was also observed that the protective layer was formed not only on the surfaces of the silver particles of the metal-based particle assembly layer, but also in gaps between the silver particles (where the core particle was exposed). It was also observed that the surface of the protective layer had recesses and projections following the recesses and projections of the metal-based particle assembly layer.

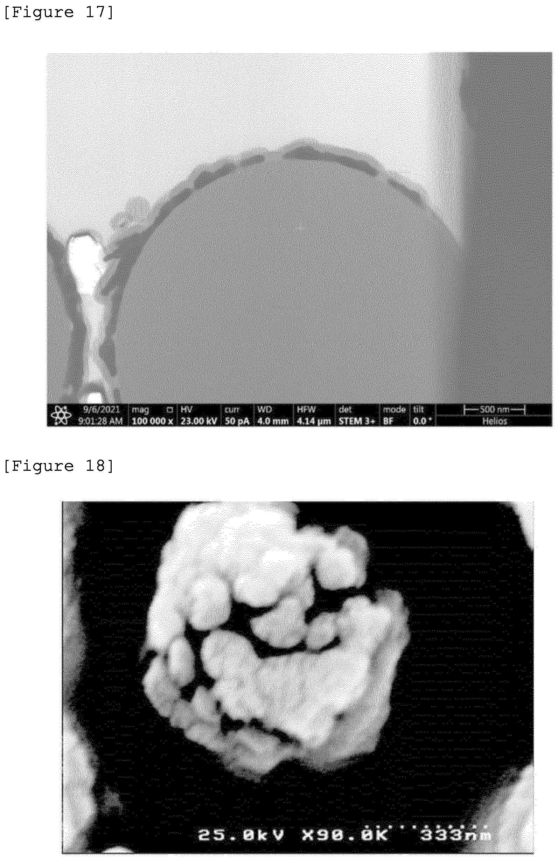

Figure 17 shows a STEM cross-sectional image (at a scale of 100000 times) of the obtained composite particles including a protective layer. The STEM cross-sectional image was obtained using the scanning transmission electron microscope "Helios G4 UX" produced by FEI Company. It was observed from this image that a protective layer having a thickness of about 60 to 70 nm was generally uniformly formed.

<Example 6>

0.75 mL of a 0.05 mol/L aqueous potassium hydroxide (KOH) solution was added dropwise to 150 mL of a 0.047 mol/L aqueous silver nitrate (AgNOs) solution, and then the mixture was stirred. Upon the addition of aqueous potassium hydroxide solution, the solution turned from clear and colorless to brown in color. To this solution, a 3.5 mol/L aqueous ethylenediamine (NH$_2$CH$_2$CH$_2$NH$_2$) solution was added dropwise in 50 μL portions with stirring, and the addition was stopped when the solution turned clear and colorless. The resulting solution is designated as a silver ion solution A.

50 mL of a 0.04 mol/L aqueous glucose solution and 25 mL of methanol were mixed to produce a reducing agent solution B. The silver ion solution A was added into a bath, and then 240 mg of silica powder (a group of spherical particles with an average particle diameter of 1.1 μm produced by JGC Catalysts and Chemicals Ltd.) as core particles were added into the bath. Then, immediately after addition of the reducing agent solution B into the bath, the solution in the bath was stirred, thereby immersing the core particles in the plating solution containing the silver ion solution A and reducing agent solution B, which initiated growth of the metal-based particles formed of silver on the surfaces of the core particles. The preparation of the plating solution and the immersion of the core particles in the plating solution were performed in an environment at 25°C. The stirring was continued at 25°C. After 28 minutes from the start of immersion, the core particles having the metal-based particle assembly layer (composite particles) were filtered off, the filtrate was then washed with a 1:1 by volume mixture of acetone and ultrapure water, and then the supernatant was removed using a centrifuge and then dried at 80°C to produce composite particles.

<Example 7>

5 mL of a 0.05 mol/L aqueous potassium hydroxide (KOH) solution was added dropwise to 666.7 mL of a 0.0024 mol/L aqueous silver nitrate (AgNOs) solution, and then the mixture was stirred. Upon the addition of aqueous potassium hydroxide solution, the solution turned from clear and colorless to brown in color. To this solution, a 3.5 mol/L aqueous ethylenediamine (NH$_2$CH$_2$CH$_2$NH$_2$) solution was added dropwise in 50 μL portions with stirring, and the addition was stopped when the solution turned clear and colorless. The resulting solution is designated as a silver ion solution A.

222.2 mL of a 0.006 mol/L aqueous glucose solution and 111.1 mL of methanol were mixed to produce a reducing agent solution B. The silver ion solution A was added into a bath, and then 46.7 mg of silica powder (a group of spherical particles with an average particle diameter of 0.55 μm produced by JGC Catalysts and Chemicals Ltd.) as core particles were added into the bath. Then, immediately after addition of the reducing agent solution B into the bath, the solution in the bath was stirred, thereby immersing the core particles in the plating solution containing the silver ion solution A and reducing agent solution B, which initiated growth of the metal-based particles formed of silver on the surfaces of the core particles. The preparation of the plating solution and the immersion of the core particles in the plating solution were performed in an environment at 25°C. The stirring was continued at 25°C. After 28 minutes from the start of immersion, the core particles having the metal-based particle assembly layer (composite particles) were filtered off, the filtrate was then washed with a 1:1 by volume mixture of acetone and ultrapure water, and then the supernatant was removed using a centrifuge and then dried at 80°C to produce composite particles.

<Example 8>

0.25 mL of a 0.05 mol/L aqueous potassium hydroxide (KOH) solution was added dropwise to 50 mL of a 0.047 mol/L aqueous silver nitrate (AgNOs) solution, and then the mixture was stirred. Upon the addition of aqueous potassium hydroxide solution, the solution turned from clear and colorless to brown in color. To this solution, a 3.5 mol/L aqueous ethylenediamine ($NH_2CH_2CH_2NH_2$) solution was added dropwise in 50 μL portions with stirring, and the addition was stopped when the solution turned clear and colorless. The resulting solution is designated as a silver ion solution A.

16.6 mL of a 0.06 mol/L aqueous glucose solution and 8.4 mL of methanol were mixed to produce a reducing agent solution B. The silver ion solution A was added into a bath, and then 362 mg of alumina powder ("Advanced Alumina AA-3" produced by Sumitomo Chemical Co., Ltd.; a group of particles with an irregular shape with an average particle diameter of 3 μm) as core particles were added into the bath. Then, immediately after addition of the reducing agent solution B into the bath, the solution in the bath was stirred, thereby immersing the core particles in the plating solution containing the silver ion solution A and reducing agent solution B, which initiated growth of the metal-based particles formed of silver on the surfaces of the core particles. The preparation of the plating solution and the immersion of the core particles in the plating solution were performed in an environment at 25°C. The stirring was continued at 25°C. After 28 minutes from the start of immersion, the core particles having the metal-based particle assembly layer (composite particles) were filtered off, the filtrate was then washed with a 1:1 by volume mixture of acetone and ultrapure water, and then the supernatant was removed using a centrifuge and then dried at 80°C to produce composite particles.

<Example 9>

A solution was obtained by dissolving 1188 μL of a 25 mass% aqueous ammonia ($NH_3$) solution in 5000 μL of ethanol. This solution was heated to 50°C, and then 24.8 mg of the composite particles obtained in Example 3 and 5.0 μL of tetraethyl orthosilicate were added with stirring at the same temperature to produce a reaction solution. The stirring of the reaction solution at 50°C was continued for 4 hours while subjecting the reaction solution to an ultrasonic treatment, and then the supernatant was removed using a centrifuge, and washed with water and dried at 80°C sequentially to produce composite particles in which a protective layer formed of SiOz was formed on the metal-based particle assembly layer. The $V_G/V_{L2}$ ratio of volume $V_G$ [cm$^3$] of tetraethyl orthosilicate to volume $V_{L2}$ [cm$^3$] of the composite particle formed of the core particle and the metal-based particle assembly layer in the reaction solution was 0.443.

A STEM cross-sectional image was obtained as in Example 5. It was observed from this image that a protective layer having a thickness of about 20 nm was generally uniformly formed.

<Example 10>

A solution was obtained by dissolving 1188 μL of a 25 mass% aqueous ammonia ($NH_3$) solution in 5000 μL of ethanol. This solution was heated to 50°C, and then 24.8 mg of the composite particles obtained in Example 3 and 15.0 μL of tetraethyl orthosilicate were added with stirring at the same temperature to produce a reaction solution. The stirring of the reaction solution at 50°C was continued for 4 hours while subjecting the reaction solution to an ultrasonic treatment, and then the supernatant was removed using a centrifuge, and washed with water and dried at 80°C sequentially to produce composite particles in which a protective layer formed of SiOz was formed on the metal-based particle assembly layer. The $V_G/V_{L2}$ ratio of volume $V_G$ [cm$^3$] of tetraethyl orthosilicate to volume $V_{L2}$ [cm$^3$] of the composite particle formed of the core particle and the metal-based particle assembly layer in the reaction solution was 1.33.

A STEM cross-sectional image was obtained as in Example 5. It was observed from this image that a protective layer having a thickness of about 85 nm was generally uniformly formed.

<Example 11>

A solution was obtained by dissolving 1188 μL of a 25 mass% aqueous ammonia ($NH_3$) solution in 5000 μL

of ethanol. This solution was heated to 50°C, and then 24.8 mg of the composite particles obtained in Example 3 and 32.4 μL of tetraethyl orthosilicate were added with stirring at the same temperature to produce a reaction solution. The stirring of the reaction solution at 50°C was continued for 4 hours while subjecting the reaction solution to an ultrasonic treatment, and then the supernatant was removed using a centrifuge, and washed with water and dried at 80°C sequentially to produce composite particles in which a protective layer formed of SiOz was formed on the metal-based particle assembly layer. The $V_G/V_{L2}$ ratio of volume $V_G$ [cm$^3$] of tetraethyl orthosilicate to volume $V_{L2}$ [cm$^3$] of the composite particle formed of the core particle and the metal-based particle assembly layer in the reaction solution was 2.87.

A STEM cross-sectional image was obtained as in Example 5. It was observed from this image that a protective layer having a thickness of about 110 nm was generally uniformly formed.

Table 2 shows, for Examples 6 to 8, the silver ion concentration in the plating solution, the type of reducing agent, the concentration of reducing agent, the ratio (in percent) of the concentration of reducing agent to the saturation concentration, the immersion time of the core particle in the plating solution, the $V_S/V_L$ ratio of volume $V_S$ [cm$^3$] of the core particle to volume $V_L$ [cm$^3$] of the plating solution, and the average height growth rate of the metal-based particles at 28 minutes from the start of deposition of the metal.

The values of X obtained based on the expression shown above for the core particles used in Examples 6 to 8 were $101 \times 10^{-7}$ for Example 6, $41 \times 10^{-7}$ for Example 7, and $110 \times 10^{-7}$ for Example 8.

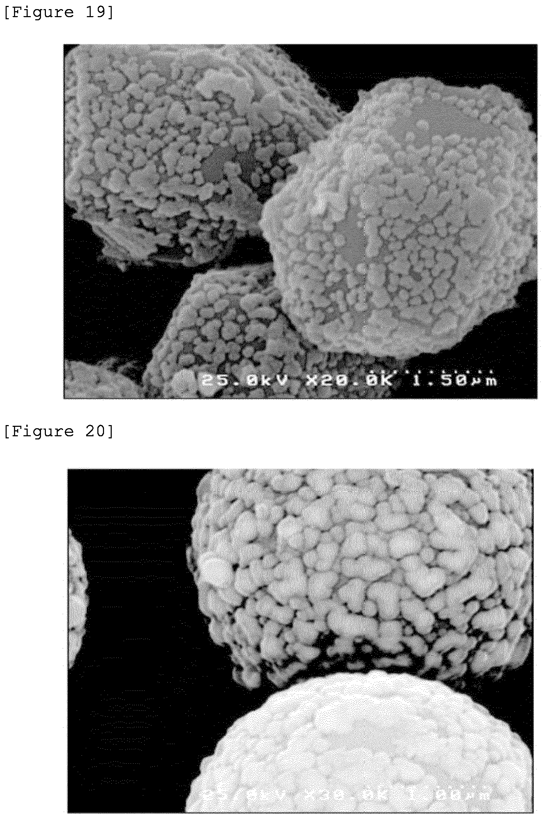

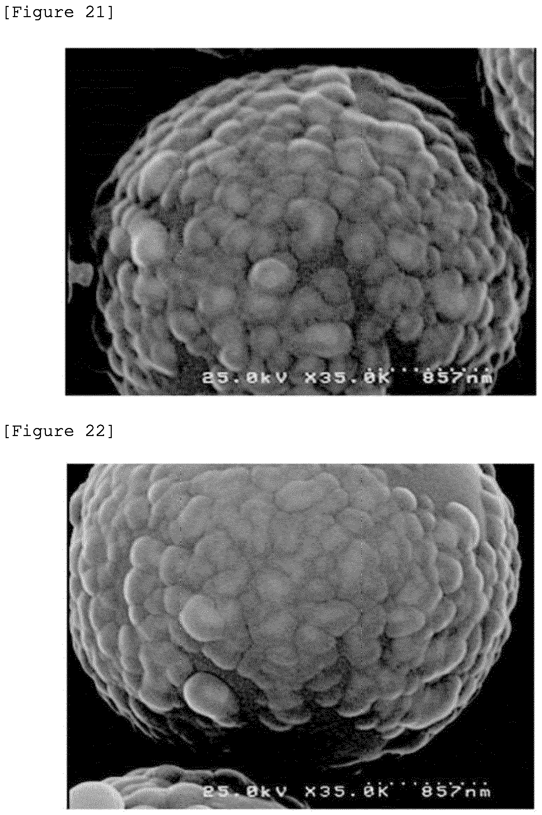

Figures 18 to 22 show SEM images of the composite particles obtained in Examples 7 to 11, respectively. From the SEM images, the average particle diameter and average interparticle distance based on the above-described definitions of the silver particles constituting the metal-based particle assembly layer covering the core particles were determined. The average height based on the above-described definition of the silver particles was also determined, based on a STEM cross-sectional image (at a scale of 100000 times). The STEM cross-sectional image was obtained using the scanning transmission electron microscope "Helios G4 UX" produced by FEI Company. The aspect ratio of the silver particles was calculated from the average particle diameter and average height obtained. These results are shown in Table 2.

[Table 2]

| | | | | Examples | | |
|---|---|---|---|---|---|---|
| | | | | 6 | 7 | 8 |
| Metal-Based Particle Growth Step | Plating Solution | Silver Ion Concentration | mol/L | 0.031 | 0.0016 | 0.031 |
| | | Type of Reducing Agent | - | Glucose | Glucose | Glucose |
| | | Concentration of Reducing Agent | mol/L | 0.0089 | 0.00133 | 0.0133 |
| | | Ratio of Concentration of Reducing Agent to Saturation Concentration | % | 0.34 | 0.05 | 0.50 |
| | Immersion Time of Core Particle in Plating Solution | | min | 28 | 28 | 28 |
| | $V_S/V_L$ | | - | 0.0005 | 0.00002 | 0.0012 |
| | Average Height Growth Rate | | nm/min | 2.5 | 3.9 | 3.0 |
| Physical Properties of Metal-Based Particle Assembly Layer | Average Particle Diameter | | nm | 235.4 | 321.4 | 187.5 |
| | Average Height | | nm | 71.3 | 110.6 | 83.0 |
| | Aspect Ratio | | - | 3.30 | 2.91 | 2.26 |
| | Average Interparticle Distance | | nm | 39.0 | 15.4 | 35.0 |

(Evaluation of Fluorescence Intensity Enhancing Effect)

For composite particles of Examples 6, 8, 9, and 11, the fluorescence intensity enhancing effect was evaluated using the same method as for the composite particles obtained in Example 3. As a result, the fluorescence intensity 3 of the composite particles of Examples 6, 8, 9, or 11 was 4.5, 1.3, 18, or 4.6 times higher than the fluorescence intensity 4, respectively. The fluorescence intensity 4 refers to the fluorescence intensity emitted from the measurement sample 4, in which the fluorescent dye, which is rhodamine B, was supported on the surfaces of the particles used as the core particles in each example, as in the evaluation for the composite particles obtained in Example 3.

Reference Signs List

1, 2: composite particle, 10: core particle, 20: metal-based particle, 30: protective layer, 50: bath, 60: plating solution, 70: substrate, 71: depression, 80: capturing portion, 90: analyte, 91: label

**Claims**

1. A composite particle comprising:

    a core particle; and
    a metal-based particle assembly layer arranged on at least a portion of a surface of the core particle,
    wherein the metal-based particle assembly layer comprises a plurality of metal-based particles arranged apart from each other.

2. The composite particle according to claim 1, wherein the plurality of metal-based particles have an average particle diameter of 5 nm or more and 1600 nm or less.

3. The composite particle according to claim 1, wherein the plurality of metal-based particles are each arranged so that an average distance between metal-based particles adjacent to each other is 1 nm or more and 150 nm or less.

4. The composite particle according to claim 1, wherein a standard deviation of the average distance is 50 nm or less.

5. The composite particle according to claim 1, wherein the surface of the core particle comprises a curved surface.

6. The composite particle according to claim 5, wherein the metal-based particle assembly layer is arranged at least on the curved surface.

7. The composite particle according to any one of claims 1 to 6, wherein the core particle has a particle diameter of 0.3 μm or more and less than 10 μm.

8. The composite particle according to any one of claims 1 to 6, wherein the metal-based particle assembly layer is arranged on an entire surface of the core particle.

9. The composite particle according to any one of claims 1 to 6, further comprising a protective layer that covers at least surfaces of the plurality of metal-based particles.

10. The composite particle according to claim 9, wherein the protective layer comprises an insulating material.

11. The composite particle according to claim 9, wherein the protective layer has a thickness of 3 nm or more and 200 nm or less.

12. The composite particle according to claim 9, wherein the protective layer covers entire surfaces of the core particle and the metal-based particles.

13. The composite particle according to any one of claims 1 to 6, which is for a sensor element.

14. A method for producing a composite particle,
    the composite particle comprising:

    a core particle; and
    a metal-based particle assembly layer arranged on at least a portion of a surface of the core particle,
    wherein the metal-based particle assembly layer comprises a plurality of metal-based particles arranged apart from each other, and
    wherein the method comprises the step of growing the metal-based particles on the surface of the core particle.

15. The method for producing a composite particle according to claim 14, further comprising, after the step of growing, a step of performing a heat treatment.

16. The method for producing a composite particle according to claim 14 or 15, further comprising, after the step of growing, a step of forming a protective layer that covers at least surfaces of the plurality of metal-based particles.

17. A composite particle comprising:

   a core particle;
   a metal-based material layer arranged on at least a portion of a surface of the core particle; and
   a protective layer that covers a surface of the metal-based material layer.

18. The composite particle according to claim 17, wherein the protective layer comprises an insulating material.

19. The composite particle according to claim 17 or 18, wherein the protective layer has a thickness of 3 nm or more and 200 nm or less.

20. A sensor element used for detecting an analyte, comprising:

   a substrate;
   a composite particle arranged on the substrate; and
   a capturing portion that is arranged on the composite particle and has a capturing substance that specifically binds to the analyte,
   wherein the composite particle comprises:

      a core particle; and
      a metal-based particle assembly layer arranged on at least a portion of a surface of the core particle,
      wherein the metal-based particle assembly layer comprises a plurality of metal-based particles arranged apart from each other.

21. The sensor element according to claim 20, wherein the composite particle further comprises a protective layer that covers at least surfaces of the plurality of metal-based particles, and
   the capturing portion is arranged on the protective layer.

[Figure 1]

[Figure 2]

[Figure 3]

[Figure 4]

[Figure 5]

[Figure 6]

25.0kV X11.0K 2.73μm

[Figure 7]

[Figure 8]

[Figure 9]

[Figure 10]

[Figure 11]

[Figure 12]

[Figure 13]

[Figure 14]

[Figure 15]

[Figure 16]

[Figure 17]

[Figure 18]

[Figure 19]

[Figure 20]

[Figure 21]

[Figure 22]

## INTERNATIONAL SEARCH REPORT

| International application No. |
|---|
| **PCT/JP2022/028198** |

**A.    CLASSIFICATION OF SUBJECT MATTER**

    ***G01N 21/64***(2006.01)i; ***B22F 9/00***(2006.01)i; ***B22F 9/24***(2006.01)i; ***C23C 18/31***(2006.01)i; ***C23C 18/44***(2006.01)i;
***C23C 28/00***(2006.01)i; ***G01N 21/41***(2006.01)i
    FI:    G01N21/64 G; B22F9/00 A; B22F9/24 E; C23C18/31 A; C23C18/44; C23C28/00 B; G01N21/41 102; G01N21/64 F

According to International Patent Classification (IPC) or to both national classification and IPC

**B.    FIELDS SEARCHED**

Minimum documentation searched (classification system followed by classification symbols)

    G01N21/00-21/83; G01N33/48-33/98

Documentation searched other than minimum documentation to the extent that such documents are included in the fields searched

    Published examined utility model applications of Japan 1922-1996
    Published unexamined utility model applications of Japan 1971-2022
    Registered utility model specifications of Japan 1996-2022
    Published registered utility model applications of Japan 1994-2022

Electronic data base consulted during the international search (name of data base and, where practicable, search terms used)

    JSTPlus/JMEDPlus/JST7580 (JDreamIII)

**C.    DOCUMENTS CONSIDERED TO BE RELEVANT**

| Category* | Citation of document, with indication, where appropriate, of the relevant passages | Relevant to claim No. |
|---|---|---|
| X | JP 2012-242162 A (FURUKAWA ELECTRIC CO., LTD.) 10 December 2012 (2012-12-10) paragraphs [0014], [0016], [0017], [0022]-[0025], fig. 2 | 1-8, 13 |
| Y | paragraphs [0014], [0016], [0017], [0022]-[0025], fig. 2 | 9-12, 14-19 |
| Y | WO 2011/096394 A1 (KONICA MINOLTA HOLDINGS, INC.) 11 August 2011 (2011-08-11) paragraphs [0027], [0030], fig. 1 | 9-12, 16-19, 21 |
| Y | JP 2008-168396 A (FUJIFILM CORP.) 24 July 2008 (2008-07-24) paragraphs [0042], [0049] | 14-16 |
| X | JP 2019-132858 A (NIPPON STEEL CHEMICAL & MATERIAL CO., LTD.) 08 August 2019 (2019-08-08) paragraphs [0033], [0050]-[0057], [0061], [0080]-[0083], fig. 1, 3 | 20 |
| Y | paragraphs [0033], [0050]-[0057], [0061], [0080]-[0083], fig. 1, 3 | 21 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 September 2022** | **20 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2022/028198**

| Patent document cited in search report | | | Publication date (day/month/year) | Patent family member(s) | Publication date (day/month/year) |
|---|---|---|---|---|---|
| JP | 2012-242162 | A | 10 December 2012 | (Family: none) | |
| WO | 2011/096394 | A1 | 11 August 2011 | (Family: none) | |
| JP | 2008-168396 | A | 24 July 2008 | (Family: none) | |
| JP | 2019-132858 | A | 08 August 2019 | US 2018/0209965 A1 paragraphs [0049], [0069]-[0081], [0085], [0107]-[0109], fig. 1, 3<br>WO 2017/010391 A1<br>EP 3321684 A1<br>EP 3869197 A1<br>CN 107850595 A<br>KR 10-2018-0030027 A<br>CN 110542761 A<br>KR 10-2020-0079559 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8271431 A

**Non-patent literature cited in the description**

- **T. FUKUURA ; M. KAWASAKI.** Long Range Enhancement of Molecular Fluorescence by Closely Packed Submicro-scale Ag Islands. *e-Journal of Surface Science and Nanotechnology,* 2009, vol. 7, 653

- Particle Measurement Technique. Nikkan Kogyo Shimbun, Ltd, 1994, 5
- **W. STOBER ; A. FINK.** *Journal of Colloid and Interface Science,* 1968, vol. 26 (1), 62-69